(19) Europäisches Patentamt
European Patent Office
Office européen des brevets

(11) Publication number: 0 444 769 A1

(12) EUROPEAN PATENT APPLICATION

(21) Application number: 91300291.1

(22) Date of filing: 16.01.91

(51) Int. Cl.5: A01N 43/647, //(A01N43/647, 43:707,35:10)

(30) Priority: 16.02.90 GB 9003553

(43) Date of publication of application: 04.09.91 Bulletin 91/36

(84) Designated Contracting States: AT BE CH DE DK ES FR GB GR IT LI LU NL SE

(71) Applicant: IMPERIAL CHEMICAL INDUSTRIES PLC
Imperial Chemical House, Millbank
London SW1P 3JF(GB)

(72) Inventor: Parham, Martin Reginald
The Nurseries, Billingbear Park
Wokingham, Berkshire(GB)
Inventor: Barton, John Edward Duncan
96 Kendrick Road
Reading, Berkshire(GB)

(74) Representative: Hardman, Carol Pauline et al
Imperial Chemical Industries PLC Legal Department: Patents PO Box 6 Bessemer Road
Welwyn Garden City Herts, AL7 1HD(GB)

# (54) Herbicidal compositions.

(57) A method for killing or controlling unwatned plant growth which method comprises applying to the plants or to the locus thereof a compound as described in EP-A-355049 and a phenoxyphenoxypropionate herbicide of the type described in EP-A-0024931 or a cyclohexanedione type herbicide of the type described in EP-A-0085529.

Compositions for use in such treatments and containers for such compositions are also described and claimed.

EP 0 444 769 A1

The present invention relates to novel herbicidal treatments and compositions for use in such treatments.

Copending European Patent Application Publication No. 355049 describes herbicidal compounds of formula (I):

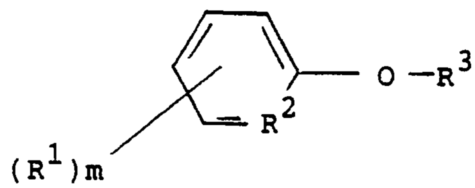

(I)

in which $R^1$ is independently selected from H, CN, $NO_2$, halogen, lower alkyl, lower haloalkyl; m is an integer of from 1 to 4; $R^2$ is N or $CR^1$ where $R^1$ is as defined above; $R^3$ is a group of formula (a), (b) or (c):

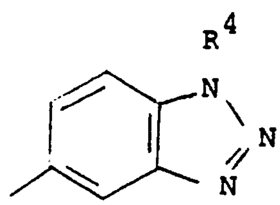

(a)

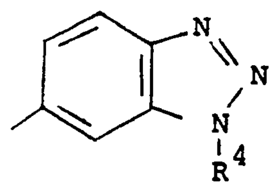

(b)

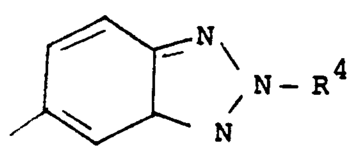

(c)

or N-oxide or quaternary salt derivatives thereof wherein $R^4$ is a group

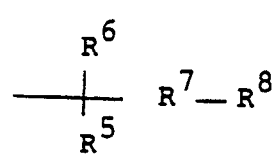

where $R^5$, $R^6$ are selected independently from H, lower alkyl, halogen or $R^5$ and $R^6$ together form $=CH_2$ or $R^5$, $R^6$ and the carbon atom to which they are attached together form a $C_{3-6}$ cycloalkyl ring; $R^7$ is $(CH_2)_n$,

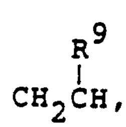

$CH=CH$,

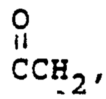

$CHOHCH_2$ or $CHOR^{10}CH_2$ where $R^9$ is lower alkyl and $R^{10}$ is lower alkyl or phenyl; and n is 0, 1 or 2; $R^8$ is $COOR^{11}$, OH, $OR^{10}$,

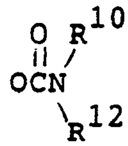 , 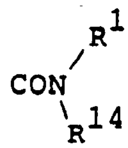 ,

$COSR^{11}$ CHO, CN, $CH=NOR^{12}$,

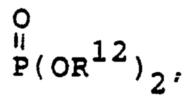

$OSO_3H$, $SO_3H$; where

$R^{10}$ is as defined hereinbefore;

$R^{11}$ is hydrogen, optionally substituted lower alkyl, optionally substituted lower alkenyl, optionally substituted lower alkynyl, $[(CH_2)_2O]_p(CH_2)_q$ $OR^{10}$, $-N=C(R^{10})_2$, optionally substituted phenyl, optionally substituted benzyl, optionally substituted cycloalkyl; p is 0, 1 or 2; q is an integer from 2 to 5 inclusive;

$R^{12}$ is H or lower alkyl;

$R^{13}$ and $R^{14}$ are selected independently from H, lower alkyl, lower cycloalkyl, alkenyl, alkynyl, $SO_2R^{10}$, $N(R^{12})_2$, $^{+}N(R^{10})_3$; additionally $R^{14}$ may be an optionally hydroxy- or phenyl-substituted alkyl radical of 1 to 4 carbon atoms, a phenyl or chlorophenyl radical, an alkoxy radical of 1 to 4 carbon atoms, or a group $-NR^{15}R^{16}$ wherein $R^{15}$ is hydrogen or alkyl of 1 to 4 carbon atoms and $R^{16}$ is hydrogen, alkyl of 1 to 4 carbon atoms, phenyl, or chlorophenyl, or the group $-NR^{13}R^{14}$ constitutes a 5 or 6-membered heterocyclic ring or alkyl quaternary derivatives; and, in the case of compounds wherein $R^8$ is a carboxyl group, salts thereof.

Some of the compounds can exist in optical isomeric forms and formula (I) include all these forms and mixtures thereof in all proportions including racemic mixtures.

Suitable optional substituents for the alkyl, alkenyl and alkynyl group of $R^{11}$ are one or more halogen atoms such as fluorine, chlorine, bromine or iodine, hydroxy and Cl-C4 alkoxy. Suitable optional substituents for the phenyl, benzyl and cycloalkyl groups are one or more halogen atoms such as fluorine, chlorine, bromine or iodine or methyl groups.

Suitable halo groups $R^1$, $R^5$ and $R^6$ include fluorine, chlorine and bromine.

Suitable haloalkyl groups $R^1$ , $R^5$ and $R^6$ include $C_{1-6}$ alkyl groups substituted with up to 6 halogen atoms selected from fluorine, chlorine and bromine. A particular haloalkyl group is trifluoromethyl. Suitable heterocyclic rings formed from $NR^{13}$ $R^{14}$ are pyrrolidine, piperidine and morpholine.

Suitable salts of compounds of formula (I) are those formed with agriculturally acceptable cations, for example sodium, potassium or quaternary ammonium salts.

Examples of quaternary ammonium ions are ions of formula $NR^{17}R^{18}R^{19}R^{20}$ where $R^{17}$, $R^{18}$, $R^{19}$ and $R^{20}$ are independently selected from hydrogen or $C_{1-10}$ alkyl optionally substituted with for example hydroxy. Suitably where any of $R^{17}$, $R^{18}$, $R^{19}$ and $R^{20}$ are optionally substituted alkyl, they contain from 1 to 4 carbon atoms.

Particular examples of $R^{17}$, $R^{18}$, $R^{19}$ and $R^{20}$, are hydrogen, ethyl and 2-hydroxyethyl.

As used herein the term "lower alkyl" includes groups containing from 1 to 10 carbon atoms, preferably from 1 to 6 carbon atoms. Similarly the terms "lower alkenyl" and "lower alkynyl" includes groups containing from 2 to 10 carbon atoms, preferably from 2 to 6 carbon atoms.

Preferably $R^1$ is selected from hydrogen, chlorine, fluorine or trifluoromethyl wherein at least one of the $R^1$ groups is other than hydrogen and at least one of the $R^1$ groups is trifluoromethyl.

Preferably $R^5$ and $R^6$ are H, lower alkyl or halogen, in particular hydrogen, methyl or fluoro.

A preferred example of the group $R^2$ is C-Cl.

Preferably $R^3$ is the group (b) as hereinbefore defined.

Preferably $R^4$ is

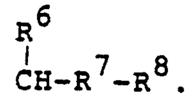

Preferably $R^7$ is $(CH_2)_n$ and n is 0 or 1, in particular 0.

Suitable groups $R^8$ are alkoxycarbonyl groups wherein the alkoxy group is straight or branched and contains up to 6 carbon atoms. In particular the group $R^8$ is $c_{1-4}$ alkoxycarbonyl, preferably ethoxycarbonyl.

A preferred sub-group of suitable compounds of formula (I) are compounds where $(R^1)_m$ is independently selected from hydrogen, halo, nitro, cyano or haloalkyl and m is an integer of from 1 to 4; $R^2$ is C-$R^1$ where $R^1$ is hydrogen, halo, nitro, cyano or haloalkyl; $R^3$ is a group of formula (b) or (c) as hereinbefore defined; $R^4$ is CH-$R^7$-$R^8$, $R^6$ is H or $C_{1-4}$ alkyl or halo, $R^7$ is $R^6$ $(CH_2)n$ where n is 0, 1 or 2, preferably 0; $R^8$ is a group $COOR^{11}$ where $R^{11}$ is as hereinbefore defined, and in particular $C_{1-4}$ alkyl, preferably methyl or ethyl.

Particular examples of compounds of formula (I) are listed in Tables I, II and III of EP-A-355049 which is hereby incorporated by reference. Additional examples of compounds of formula (I) are described in corresponding EP-A-0367242 and these examples also are included herein by reference.

These compounds are active as herbicides.

Copending EP-A-355049 discloses that the compounds of formula (I) can be mixed with a wide range of other herbicides including phenoxyphenoxypropionate herbicides and cyclohexanedione herbicides.

The Applicants have now found that the compounds of formula (I) can be advantageously mixed with particular groups of other herbicides.

Additional herbicidal compounds are described in EP-A-0024931.

This patent application describes compounds of formula (II):

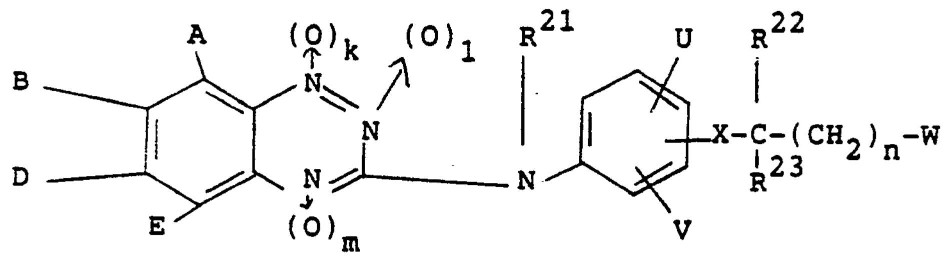

(II)

or a salt thereof wherein:

A,B,D,E,U and V are independently chosen from the group consisting of hydrogen, halogen, nitro, cyano, thiocyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_3$ to $C_7$ cycloalkyl, $C_1$ to $C_6$ alkoxy, $C_1$ to $C_6$ haloalkoxy, $C_1$ to $C_6$ alkylthio, $C_1$ to $C_6$ alkylsulfinyl, $C_1$ to $C_6$ alkylsulfonyl, $C_1$ to $C_6$ haloalkylsulfinyl, $C_1$ to $C_6$ haloalkylsulfonyl, sulfo, $C_1$ to $C_6$ alkoxysulfonyl, sulfamoyl, N-($C_1$ to $C_6$ alkyl)sulfamoyl, N-N-di($C_1$ to $C_6$ alkyl)sulfamoyl, carboxy, ($C_1$ to $C_6$ alkoxy)-carbonyl, carbamoyl, N-($C_1$ to $C_6$ alkyl) carbamoyl, N-N di($C_1$ to $C_6$ alkyl)carbamoyl, phenyl, phenoxy, phenylthio, and the groups substituted phenyl, substituted phenoxy and substituted phenylthio wherein in each group the phenyl ring is substituted with from 1 to 3 substituents chosen from the group consisting of halogen, $C_1$ to $C_6$ alkyl, $C_1$ to $C_6$ haloalkyl, $C_1$ to $C_6$ alkoxy, nitro and cyano;

$R^{21}$ is chosen from the group consisting of hydrogen, $C_1$ to $C_{10}$ alkyl, $C_2$ to $C_{10}$ alkenyl, $C_2$ to $C_{10}$ alkynyl, $C_2$ to $C_{10}$ alkoxyalkyl, cyanomethylene, ($C_1$ to $C_6$ alkoxy)carbonylmethylene, $C_1$ to $C_{10}$ haloalkyl, formyl, $C_2$ to $C_{10}$ alkanoyl, phenyl, benzyl, benzoyl, and the groups phenyl, benzyl and benzoyl wherein in each group the phenyl ring is substituted with from 1 to 3 substituents chosen from the group consisting of halogen, $C_1$ to $C_6$ alkyl, $C_1$ to $C_6$ haloalkyl, $C_1$ to $C_6$ alkoxy, nitro and cyano;

$R^{22}$ is chosen from the group consisting of hydrogen, $C_1$ to $C_6$ alkyl, $C_2$ to $C_6$ alkenyl, $C_2$ to $C_6$ alkoxyalkyl, $C_1$ to $C_6$ haloalkyl, acetyl, propionyl and $C_2$ to $C_6$ alkoxycarbonyl;

$R^{23}$ is chosen from the group consisting of hydrogen, $C_1$ to $C_6$ alkyl, $C_2$ to $C_6$ alkenyl, $C_2$ to $C_6$ alkoxyalkyl and $C_1$ to $C_6$ haloalkyl, or $R^2$ and $R^3$ together may form a methylene, ethylidene, propylidene or isopropylidene group;

W is chosen from the group consisting of cyano, thiocarbamoyl,

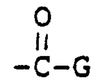

and $CH_2Z$ wherein: G is chosen from the group consisting of hydroxy, mercapto, $C_1$ to $C_{10}$ alkoxy, $C_1$ to $C_{10}$ haloalkoxy, $C_2$ to $C_{10}$ alkenyloxy, $C_2$ to $C_{10}$ alkynyloxy, $C_1$ to $C_{10}$ alkylthio, $C_2$ to $C_{10}$ alkenylthio, $C_2$ to $C_{10}$ alkynylthio, $C_3$ to $C_7$ cycloalkoxy, $C_3$ to $C_7$ cycloalkoxy substituted with 1 or 2 $C_1$ to $C_4$ alkyl groups, phenoxy, phenylthio, benzyloxy, benzylthio, the group $C_1$ to $C_6$ alkoxy substituted with a substituent chosen from the group consisting of $C_1$ to $C_6$ alkoxy, amino, ammonio, cyano, N-($C_1$ to $C_6$ alkyl)amino, N,N-di($C_1$ to $C_6$ alkyl)amino and N,N,N-tri-($C_1$ to $C_6$ alkyl) ammonio, the groups phenoxy, phenylthio, benzyloxy and benzylthio wherein in each group the phenyl ring is substituted with from 1 to 3 substituents chosen from the group consisting of halogen, nitro, cyano, $C_1$ to $C_6$ alkyl, $C_1$ to $C_6$ haloalkyl and $C_1$ to $C_6$ alkoxy, the group OM wherein M is the cation of an inorganic or organic base, the group $-NHSO_2R^{24}$ wherein $R^{24}$ is chosen from $C_1$ to $C_{10}$ alkyl and $C_{16}$ to $C_{10}$ haloalkyl, and the group $-NR^{25}R^{26}$ wherein $R^{25}$ and $R^{26}$ are independently chosen from the group consisting of hydrogen, $C_1$ to $C_6$ alkyl, $C_1$ to $C_6$ hydroxyalkyl, $C_1$ to $C_6$ haloalkyl, phenyl and benzyl, or $R^{25}$ and $R^{26}$ together form a heterocyclic ring, and the group-O-N = $R^{27}$ wherein $R^{27}$ is a $C_1$ to $C_{10}$ alkylidene group; and z is chosen from halogen, hydroxy, mercapto, $C_1$ to $C_{10}$ alkoxy, $C_1$ to $C_{10}$ haloalkoxy, $C_1$ to $C_{10}$ alkylthio, and the group $NR^{25}R^{26}$ wherein $R^{25}$ and $R^{26}$ are as hereinbefore defined; x is chosen from oxygen and sulfur;

k, l and m are independently chosen from 0 and 1 provided that k + l + m is 0, 1 or 2; and n is 0, 1 or 2.

The compounds of formula II wherein $R^{22}$ and $R^{23}$ are not the same, are optically active and the present invention also includes the individual stereo isomers of such compounds, and mixtures of those stereo isomers in addition to the racemic mixture of stereo isomers.

Preferred sub-groups of formula (II) are described in EP-A-0024931 which is incorporated herein by reference.

For the present purposes, particular suitable compounds of formula (II) are those where A, D and E are hydrogen, B is halogen such as chlorine, k is 1 and l and m are 0, $R^{21}$ is lower alkyl such as methyl, U and V are hydrogen, X is oxygen, $R^{22}$ and $R^{23}$ are independently selected from hydrogen or lower alkyl such as methyl, n is 0 and W is a group

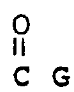

where G is $C_1$ to $C_{10}$ alkoxy.

Yet more herbicidal compounds, this time falling within the class of cyclohexanedione herbicides are described in EP-A-0085529.

This publication discloses herbicide compounds of formula (III):

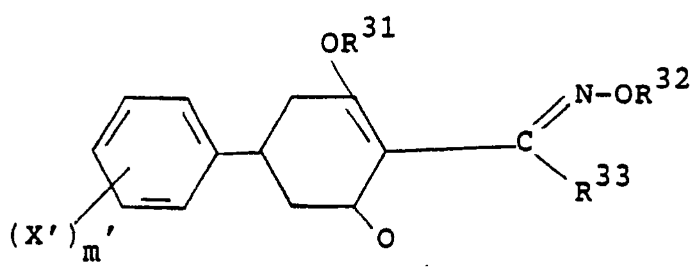

(III)

wherein:

X', which may be the same or different, are selected from the group consisting of: halogen; nitro; cyano; $C_1$ to $C_6$ alkyl; $C_1$ to $C_6$ alkyl substituted with a substituent selected from the group consisting of halogen, nitro, hydroxy, $C_1$ to $C_6$ alkoxy and $C_1$ to $C_6$ alkylthio; $C_2$ to $C_6$ alkenyl; $C_2$ to $C_6$ alkynyl; hydroxy; $C_1$ to $C_6$ alkoxy; $C_1$ to $C_6$ alkoxy substituted with a substituent selected from halogen and $C_1$ to $C_6$ alkoxy; $C_2$ to $C_6$ alkenyloxy; $C_2$ to $C_6$ alkynyloxy; $C_2$ to $C_6$ alkanoyloxy; ($C_1$ to $C_6$ alkoxy)carbonyl; $C_1$ to $C_6$ alkylthio; $C_1$ to $C_6$ alkylsulfinyl; $C_1$ to $C_6$ alkylsulfonyl; sulfamoyl; N-($C_1$ to $C_6$ alkyl)sulfamoyl; N,N-di($C_1$ to $C_6$ alkyl)-

sulfamoyl; benzyloxy; substituted benzyloxy wherein the benzene ring is substituted with from one to three substituents selected from the group consisting of halogen, nitro, $C_1$ to $C_6$ alkyl, $C_1$ to $C_6$ alkoxy and $C_1$ to $C_6$ haloalkyl; the group $NR^{38}R^{39}$ wherein $R^{38}$ and $R^{39}$ are independently selected from the group consisting of hydrogen, $C_1$ to $C_6$ alkyl, $C_2$ to $C_6$ alkanoyl, benzoyl and benzyl; the groups formyl and $C_2$ to $C_6$ alkanoyl and the oxime, imine and Schiff base derivatives thereof; and at least one of X is not selected from the group consisting of halogen, $C_1$ to $C_6$ alkyl and $C_1$ to $C_6$ alkoxy;

$R^{31}$ is selected from the group consisting of: hydrogen; $C_1$ to $C_6$ alkyl; $C_2$ to $C_6$ alkenyl; $C_2$ to $C_6$ alkynyl; substituted $C_1$ to $C_6$ alkyl wherein the alkyl group is substituted with a substituent from the group consisting of $C_1$ to $C_6$ alkoxy, $C_1$ to $C_6$ alkylthio, phenyl and substituted phenyl wherein the benzene ring is substituted with from one to three substituents selected from the group consisting of halogen, nitro, cyano, $C_1$ to $C_6$ alkyl, $C_1$ to $C_6$ haloalkyl, $C_1$ to $C_6$ alkoxy and $C_1$ to $C_6$ alkylthio; $C_1$ to $C_6$ (alkyl) sulfonyl; benzenesulfonyl;

substituted benzenesulfonyl wherein the benzene ring is substituted with from one to three substituents selected from the group consisting of halogen, nitro, cyano, $C_1$ to $C_6$ alkyl, $C_1$ to $C_6$ haloalkyl, $C_1$ to $C_6$ alkoxy and $C_1$ to $C_6$ alkylthio; and acyl group; and an inorganic or organic cation;

$R^{32}$ is selected from the group consisting of: $C_1$ to $c_6$ alkyl; $C_2$ to $C_6$ alkenyl; $C_2$ to $C_6$ haloalkenyl; $C_2$ to $C_6$ alkynyl; $C_2$ to $C_6$ haloalkynyl; substituted $C_1$ to $C_6$ alkyl wherein the alkyl group is substituted with a substituent selected from the group consisting of halogen, $C_1$ to $C_6$ alkoxy, $C_1$ to $C_6$ alkylthio, phenyl and substituted phenyl wherein the benzene ring is substituted with from one to three substituents selected from the group consisting of halogen, nitro, cyano, $C_1$ to $C_6$ alkyl, $C_1$ to $C_6$ haloalkyl, $C_1$ to $C_6$ alkoxy and $C_1$ to $C_6$ alkylthio;

$R^{37}$ is selected from the group consisting of: $C_1$ to $C_6$ alkyl; $C_1$ to $C_6$ fluoroalkyl; $C_2$ to $C_6$ alkenyl; $C_2$ to $C_6$ alkynyl; and phenyl; and

m is an integer from 3 to 5.

Preferred sub groups of compounds of formula (III) are described in EP-A-0085529 which is incorporated herein by reference.

For the present purpose, a particularly preferred sub group of compounds of formula (III) are those of formula (IIIA):

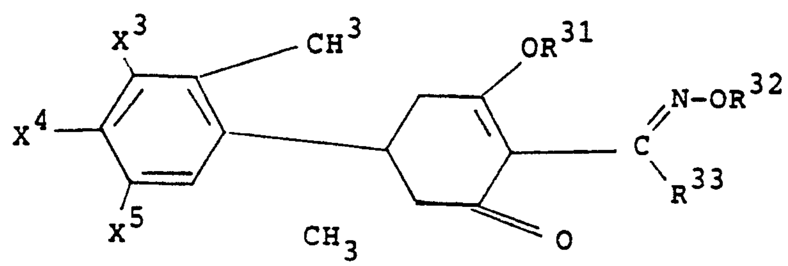
(IIIA)

wherein:

$X^3$ is selected from the group consisting of methylmercapto, nitromethyl, methoxymethyl, ethoxymethyl, methylsulfinyl, methylsulfonyl, acetyl, propionyl, sulfamoyl and N,N-dimethylsulfamoyl;

$X^4$ is selected from hydrogen and methyl;

$X^5$ is selected from hydrogen, methyl and ethyl;

$R^{31}$ is selected from hydrogen, sodium and potassium;

$R^{32}$ is selected from ethyl and allyl; and

$R^{33}$ is selected from ethyl and n-propyl.

According to the present invention there is provided a method of killing or controlling unwanted plant growth, which method comprises applying to the plant or the locus thereof a compound of formula (I) as hereinbefore defined and a compound of formula (II) as hereinbefore defined or a compound of formula (III) as hereinbefore defined.

The compounds of formula (I) and formula (II) or formula (III) may be applied directly to the plant (post-emergence application) or to the soil before the emergence of the plant (pre-emergence application). They are particularly useful when applied post-emergence.

The compounds of formula (I) and formula (II) or formula (III) may be applied individually to inhibit the growth of, severely damage, or kill plants but are preferably used in the form of an admixed composition comprising compounds of formula (I) and formula (II) or formula (III) in admixture with a carrier comprising a solid or liquid diluent

Therefore, in yet a further aspect the invention provides plant growth inhibiting, plant damaging, or plant killing compositions comprising a compound of formula (I) as hereinbefore defined, and a compound of formula (II) or compound of formula (III) and an inert carrier or diluent.

The compounds of formula (I) and formula (II) or (III) are formulated in a single composition. However they may be formulated separately for application sequentially or for admixture prior to administration, for example after dissolution in water. They may in fact be presented in a two pack-container, one compartment of which containing a composition comprising a compound of formula (I) in combination with a solid or liquid diluent with or without adjuvant and the second compartment contains a composition comprising a compound of formula (II) or a compound of formula (III) in combination with a solid or liquid diluent and optionally also an adjuvant. One component maybe in the form of a solid formulation for example as granule, while the other may be in the form of a liquid concentrate.

The contents of the two compartments can then be admixed, for example by dissolving both in aqueous solution prior to administration.

Compositions containing compounds of formulae (I) and/or (II) or (III) include both dilute compositions, which are ready for immediate use, and concentrated compositions, which require to be diluted before use, usually with water. Preferably the compositions contain from 0.01% to 90% by weight of the active ingredient. Dilute compositions ready for use preferably contain from 0.01% to 2% of active ingredient, while concentrated compositions may contain from 20 to 90% of active ingredient, although from 20 to 70% is usually preferred.

The solid compositions may be in the form of granules, or dusting powders wherein the active ingredient is mixed with a finely divided solid diluent, e.g. kaolin, bentonite, kieselguhr, dolomite, calcium carbonate, talc, powdered magnesia, Fuller's earth and gypsum. They may also be in the form of dispersible powders or grains, comprising a wetting agent to facilitate the dispersion of the powder or grains in liquid. Solid compositions in the form of a powder may be applied as foliar dusts.

Liquid compositions may comprise a solution or dispersion of an active ingredient in water optionally containing a surface-active agent, or may comprise a solution or dispersion of an active ingredient in a water-immiscible organic solvent which is dispersed as droplets in water.

Surface-active agents may be of the cationic, anionic, or non-ionic type or mixtures thereof. Optionally they are mixed with other adjuvants such as the emulsifiable crop oils. The cationic agents are, for example, quaternary ammonium compounds (e.g. cetyltrilmethylammonium bromide). Suitable anionic agents are soaps; salts of aliphatic mono ester of sulphuric acid, for example sodium lauryl sulphate; and salts of sulphonated aromatic compounds, for example sodium dodecylbenzenesulphonate, sodium, calcium, and ammonium lignosulphonate, butylnaphthalene sulphonate, and a mixture of the sodium salts of diisopropyl and triisopropylnaphthalenesulphonic acid. Suitable non-ionic agents are the condensation products of ethylene oxide with fatty alcohols such as oleyl alcohol and cetyl alcohol, or with alkylphenols such as octyl- or nonylphenol (e.g. Agral 90) or octyl-cresol. Other non-ionic agents are the partial esters derived from long chain fatty acids and hexitol anhydrides, for example sorbitan monolaurate; the condensation products of the partial ester with ethylene oxide; and the lecithins; silicone surface active agents (water soluble surface active agents having a skeleton which comprises a siloxane chain e.g. Silwet L77). Oil surfactant blends are also suitable adjuvants and a suitable mixture in mineral oil is Atplus 411F.

The aqueous solutions or dispersions may be prepared by dissolving the active ingredient in water or an organic solvent optionally containing wetting or dispersing agent(s) and then, when organic solvents are used, adding the mixture so obtained to water optionally containing wetting or dispersing agent(s). Suitable organic solvents include, for example, ethylene di-chloride, isopropyl alcohol, propylene glycol, diacetone alcohol, toluene, kerosene, methylnaphthalene, the xylenes and trichloroethylene.

The compositions for use in the form of aqueous solutions or dispersions are generally supplied in the form of a concentrate containing a high proportion of the active ingredient, and the concentrate is then diluted with water before use. The concentrates are usually required to withstand storage for prolonged periods and after such storage, to be capable of dilution with water to form aqueous preparations which remain homogeneous for a sufficient time to enable them to be applied by conventional spray equipment. Concentrates conveniently contain 20-90%, preferably 20-70%, by weight of the active ingredient(s). Dilute preparations ready for use may contain varying amounts of the active ingredient(s) depending upon the intended purpose; amounts of 0.01% to 10.0% and preferably 0.01% to 2%, by weight of active ingredient-(s) are normally used.

A preferred form of concentrated composition comprising the active ingredient which has been finely divided and which has been dispersed in water in the presence of a surface-active agent and a suspending agent. Suitable suspending agents are hydrophilic colloids and include, for example, polyvinylpyrrolidone and sodium carboxymethylcellulose, and the vegetable gums, for example gum acacia and gum tragacanth.

Preferred suspending agents are those which impart thixotropic properties too, and increase the viscosity of the concentrate. Examples of preferred suspending agents include hydrated colloidal mineral silicates, such as montmorillonite, beidellite, nontronite, hectorite, saponite, and saucorite. Bentonite is especially preferred. Other suspending agents include cellulose derivatives and polyvinyl alcohol.

The rate of application of the compounds of the invention will depend on a number of factors including, for example, the compound of formulae (I), and (II) or (III) chosen for use, the identity of the plants whose growth is to be inhibited, the formulations selected for use and whether the compound is to be applied for foliage or root uptake. As a general guide, however, an application rate of from 0.001 to 1 kilograms total a.i. per hectare is suitable while from 0.010 to 0.5 kilograms total a.i. per hectare may be preferred and most preferably 0.01 to 0.25 kilograms total a.i. per hectare is used. The ratio of the compound of formula (I) to the compound of formula (II) or (III) applied is suitably in the range of from 1:10 to 10:1, preferably 1:5 to 5:1. Obviously the ratio, as with the rate itself will vary depending upon environmental factors, the particular species to be treated, the technical effect desired and the compounds employed. For instance, in the tests shown hereinafter, a ratio of 3:1 is preferred.

The treatment of the invention can be used in association with the application of another biologically active molecule such as another herbicide, fungicide, insecticide optionally in the presence of an insecticide synegist, or a plant growth regulator. The other molecule may be incorporated in the composition of the invention or maybe admixed with a prepared composition, or applied separately before or after the treatment of the invention.

The other herbicide may be any herbicide not having the formula (I). It will generally be a herbicide having a complementary action in the particular application.

Examples of useful complementary herbicides include:

A. benzo-2,1,3-thiadiazin-4-one-2,2-dioxides such as bentazone;

B. hormone herbicides, particularly the phenoxy alkanoic acids such as MCPA, MCPA-thioethyl, dichlorprop, 2,4,5-T, MCPB, 2,4-D, 2,4-DB, mecoprop, trichlopyr, clopyralid, and their derivatives (eg. salts, esters and amides);

C. 1,3 dimethylpyrazole derivatives such as pyrazoxyfen, pyrazolate and benzofenap;

D. Dinitrophenols and their derivatives (eg. acetates) such as dinoterb, dinoseb and its ester, dinoseb acetate;

E. dinitroaniline herbicides such as dinitramine, trifluralin, ethalflurolin, pendimethalin, oryzalin;

F. arylurea herbicides such as diuron, flumeturon, metoxuron, neburon, isoproturon, chlorotoluron, chloroxuron, linuron, monolinuron, chlorobromuron, daimuron, methabenzthiazuron;

G. phenylcarbamoyloxyphenylcarbamates such as phenmedipham and desmedipham;

H. 2-phenylpyridazin-3-ones such as chloridazon and norflurazon;

I. uracil herbicides such as lenacil, bromacil and terbacil;

J. triazine herbicides such as atrazine, simazine, aziprotryne, cyanazine, prometryn, dimethametryn, simetryne, and terbutryn;

K. phosphorothioate herbicides such as piperophos, bensulide, and butamifos;

L. thiolcarbamate herbicides such as cycloate, vernolate, molinate, thiobencarb, butylate *, EPTC *, tri-allate, di-allate, esprocarb, tiocarbazil, pyridate, and dimepiperate;

M. 1,2,4-triazin-5-one herbicides such as metamitron and metribuzin;

N. benzoic acid herbicides such as 2,3,6-TBA, dicamba and chloramben;

O. anilide herbicides such as pretilachlor, butachlor, alachlor, propachlor, propanil, metazachlor, metolachlor, acetochlor, and dimethachlor;

P. dihalobenzonitrile herbicides such as dichlobenil, bromoxynil and ioxynil;

Q. haloalkanoic herbicides such as dalapon, TCA and salts thereof;

R. diphenylether herbicides such as lactofen, fluroglycofen or salts or ester thereof, nitrofen, bifenox, aciflurofen and salts and esters thereof, oxyfluorfen, fomesafen, chlornitrofen and chlomethoxyfen;

S. phenoxyphenoxypropionate herbicides such as diclofop and esters thereof such as the methyl ester, fluazifop and esters thereof, haloxyfop and esters thereof, quizalofop and esters thereof and fenoxaprop and esters thereof such as the ethyl ester;

T. cyclohexanedione herbicides such as alloxydim and salts thereof, sethoxydim, cycloxyidim, tralkoxydim, and clethodim;

U. sulfonyl urea herbicides such as chlorosulfuron, sulfometuron, metsulfuron and esters thereof; benzsulfuron and esters thereof such as DPX-M6313, chlorimuron and esters such as the ethyl ester

* These compounds are preferably employed in combination with a safener such as dichlormid.

thereof pirimisulfuron and esters such as the methyl ester thereof, 2-[3-(4-methoxy-6-methyl-1,3,5-triazinzyl)-3-methylureidosulphonyl) benzoic acid esters such as the methyl ester thereof (DPX-LS300) and pyrazosulfuron;

V. imidazolidinone herbicides such as imazaquin, imazamethabenz, imazapyr and isopropylammonium salts thereof, imazethapyr;

W. arylanilide herbicides such as flamprop and esters thereof, benzoylprop-ethyl, diflufenican;

X. amino acid herbicides such as glyphosate and glufosinate and their salts and esters, sulphosate and bialaphos;

Y. organoarsenical herbicides such as monosodium methanearsonate (MSMA);

Z. herbicidal amide derivative such as napropamide, propyzamide, carbetamide, tebutam, bromobutide, isoxaben, naproanilide and naptalam;

AA. miscellaneous herbicides including ethofumesate, cinmethylin, difenzoquat and salts thereof such as the methyl sulphate salt, clomazone, oxadiazon, bromofenoxim, barban, tridiphane, flurochloridone, quinchlorac, dithiopyr and mefanacet;

BB. Examples of useful contact herbicides include:

bipyridylium herbicides such as those in which the active entity is paraquat and those in which the active entity is diquat;

The following Examples illustrate the invention.

EXAMPLE 1

Specific combinations of compounds of formula (I) with compounds of formula (II) and (III) include the following:

| | Compounds of formula (I) | Compounds of formula (II) or (III) |
|---|---|---|
| a) | Ethyl 2-[6-(2-chloro-6-fluoro-4-trifluoromethyl phenoxy)benzotriazol-1-yl]propionate. | 5-(3-acetyl-2,4,6-trimethylphenyl)-2-[1-(ethoxyimino)-propyl]-3-hydroxycyclohex-2-en-1-one. |
| b) | Ethyl 2-[6-(2-chloro-6-fluoro-4-trifluoromethyl phenoxy)benzotriazol-1-yl]propionate. | 2-[1-(ethoxyimino)propyl]-3-hydroxy-5-(2,4,6-trimethyl-3-butyrylphenyl)cyclohex-2-enone |
| c) | Methyl 2-[6-(2-chloro-6-fluoro-4-trifluoromethyl phenoxy)benzotriazol-1-yl]propionate. | 2-[1-(ethoxyimino)propyl]-3-hydroxy-5-(2,4,6-trimethyl-3-butyrylphenyl)cyclohex-2-enone. |
| d) | Methyl 2-[6-(2-chloro-6-fluoro-4-trifluoromethyl phenoxy)benzotriazol-1-yl]propionate. | 5-(3-acetyl-2,4,6-trimethylphenyl)-2-[1-(ethoxyimino)-propyl]-3-hydroxycyclohex-2-en-1-one. |
| e) | 2-[6-(2-chloro-6-fluoro-4-trifluoromethyl phenoxy)benzotriazol-1-yl] propionic acid. | 5-(3-acetyl-2,4,6-trimethylphenyl)-2-[1-(ethoxyimino)-propyl]-3-hydroxycyclohex-2-en-1-one. |

| | Compounds of formula (I) | Compounds of formula (II) or (III) |
|---|---|---|
| f) | 2-[6-(2-chloro-6-fluoro-4-trifluoromethyl phenoxy)benzotriazol-1-yl] propionic acid. | 2-[1-(ethoxyimino)propyl]-3-hydroxy-5-(2,4,6-trimethyl-3-butyrylphenyl)cyclohex-2-enone. |
| g) | Ethyl 2-[6-(2,6-dichloro-4-trifluoromethyl phenoxy)benzotriazol-1-yl]propionate. | 5-(3-acetyl-2,4,6-trimethylphenyl)-2-[1-(ethoxyimino-propyl]-3-hydroxycyclohex-2-enone. |
| h) | Ethyl 2-[6-(2,6-dichloro-4-trifluoromethyl phenoxy)benzotriazol-1-yl]propionate. | 2-[1-(ethoxyimino)propyl]-3-hydroxy-5-(2,4,6-trimethyl-3-butynylphenyl)cyclohex-2-enone. |
| i) | Ethyl 2-[6-(2,6-difluoro-4-trifluoromethyl phenoxy)benzotriazol-1-yl]propionate. | 2-[1-(ethoxyimino)propyl]-3-hydroxy-5-(2,4,6-trimethyl-3-butylphenyl)cyclohex-2-enone. |
| j) | Ethyl 2-[6-(2,6-difluoro-4-trifluoromethyl phenoxy)benzotriazol-1-yl]propionate. | 5-(3-acetyl-2,4,6-trimethylphenyl)-2-[1-(ethoxyimino)-propyl]-3-hydroxycyclohex-2-en-1-one. |

| | Compounds of formula (I) | Compounds of formula (II) or (III) |
|---|---|---|
| k) | Ethyl 2-[6-(2-chloro-6-fluoro-4-trifluoromethyl phenoxy)benzotriazol-1-yl]propionate. | Methyl 2-(4-[N-methyl-N-(7-chloro-1-oxide-1,2,4-benzotriazin-3-yl)amino] phenoxy)propionate |
| l) | Ethyl 2-[6-(2-chloro-6-fluoro-4-trifluoromethyl phenoxy)benzotriazol-1-yl]propionate. | Iso propyl(R)-2-[4-(7-chloro-1-oxide -1,2,4-benzotriazin-3-ylmethylamino) phenoxy]propionate. |
| m) | Methyl 2-[6-(2-chloro-6-fluoro-4-trifluoromethyl phenoxy)benzotriazol-1-yl]propionate. | Iso propyl(R)-2-[4-(7-chloro-1-oxide -1,2,4-benzotriazin-3-ylmethylamino) phenoxy]propionate. |
| n) | Methyl 2-[6-(2-chloro-6-fluoro-4-trifluoromethyl phenoxy)benzotriazol-1-yl]propionate. | Methyl 2-(4-[N-methyl-N-(7-chloro-1-oxide-1,2,4-benzotriazin-3-yl)amino] phenoxy)propionate. |
| o) | 2-[6-(2-chloro-6-fluoro-4-trifluoromethyl phenoxy)benzotriazol-1-yl] propionic acid. | Methyl 2-(4-[N-methyl-N-(7-chloro-1-oxide-1,2,4-benzotriazin-3-yl)amino] phenoxy)propionate. |

| | Compounds of formula (I) | Compounds of formula (II) or (III) |
|---|---|---|
| p) | 2-[6-(2-chloro-6-fluoro-4-trifluoromethyl phenoxy)benzotriazol-1-yl] propionic acid. | Iso propyl(R)-2-[4-(7-chloro-1-oxide-1 2,4-benzotriazin-3-ylmethylamino) phenoxy]propionate. |
| q) | Ethyl 2-[6-(2,6-dichloro-4-trifluoromethyl phenoxy)benzotriazol-1-yl]propionate. | Methyl 3-(4-[N-methyl-N-(7-chloro-1-oxide-1,2,4-benzotriazin-3-yl)amino phenoxy]propionate. |
| r) | Ethyl 2-[6-(2,6-dichloro-4-trifluoromethyl phenoxy)benzotriazol-1-yl]propionate. | Iso propyl(R)-2-[4-(7-chloro-1-oxide-1 2,4-benzotriazin-3-ylmethylamino) phenoxy)propionate. |
| s) | Ethyl 2-[6-(2,6-dichloro-4-trifluoromethyl phenoxy)benzotriazol-1-yl]propionate. | Iso propyl(R)-2-(4-7-chloro-1-oxide-1 2,4 benzotrizin-3-ylmethyl amino)phenoxy propionate. |
| t) | Ethyl 2-[6-(2,6-difluoro-4-trifluoromethyl phenoxy)benzotriazol-1-yl]propionate. | Methyl 2-(4-[N-methyl-N-(7-chloro-1-oxide-1,2,4-benzotriazin-3-yl)amino] phenoxy)propionate. |

EXAMPLE 2

The biological efficiency of the invention was demonstrated in the following tests:

Compound 1 - Ethyl 2-[6-(2-chloro-6-fluoro-4-trifluoromethylphenoxy)benzotriazol-1-yl]-propionate

Compound 2 - 2-[1-(ethoxyimino)propyl]-3-hydroxy-5-(2,4,6-trimethyl-3-butyrylphenyl)cyclohex-2-

enone

Compounds 1 and 2 and mixtures of thereof in the ratios 0:1, 1:0, 1:1, 1:3 and 3:1 were applied to the following established annuals:

| | |
|---|---|
| Grasses - | Eleusine indica (EI) |
| | Sorghum halepense (SH) |
| | Digitaria sanguinalis (DG) |
| | Setaria viridis (ST) |
| | Elymus repens (AG) |
| | Lolium perenne (LL) |
| Sedge - | Cyperus rotundus (CN) |
| BL Weeds - | Euphorbia heterophylla (EH) |
| | Polygonum aviculare (PI) |
| | Matricaria perforata (TM) |
| | Conyza canadensis (ECA) |

The compounds were dissolved to form a 5% solution in methylcyclohexanone containing 21.8g 1 'Span 80' and 78.2g/1 Tween 20 surfactants. This solution was emulsified in water to give the required concentration of chemical in the spray solution. This solution was sprayed with 1%. 'Atplus' 411F at 2001/ha using a fan-jet mounted in a laboratory track sprayer.

Plants were grown in a glasshouse in 7cm pots of a proprietary soil based compost (John Innes No. 1). At the time of spraying they had at least 6 full leaves and ranged from 5-12cm in height. After spraying the plants were held in a cool glasshouse with temperatures of 16-20° C for the temperate climate species and a glasshouse at 20-24° C for the warm climate species. Daylength was maintained at 14 hours using mercury vapour lamps.

The plants were assessed at intervals during the month after spraying to record visual symptoms of the chemical effects. A scale of 0-10 was used, where 0 = nr effect, 10 = total plant death.

The results are shown in Table I attached.

TABLE I

6 DAT RESULTS

| Compound | RATE g/ha | RATE g/ha | DG | SH | ST | AG | LL | CN | EH | PI | EI | TM | ECA |
|---|---|---|---|---|---|---|---|---|---|---|---|---|---|
| 1 | 0.78125 | | | 5.0 | | | | | 13.0 | 9.7 | 7.7 | | |
| above | 1.5625 | 0 | 16.7 | 7.3 | | | | | 28.3 | 15.7 | 19.0 | 54.0 | |
| | 3.125 | 0 | 30.0 | 18.3 | 33.3 | 30.0 | | | 51.7 | 50.7 | 42.7 | 69.0 | |
| | 6.25 | 0 | 41.7 | 40.0 | 46.7 | 43.3 | 34.0 | | 75.0 | 67.0 | 56.0 | 79.3 | |
| | 12.5 | 0 | 73.7 | 71.7 | 69.0 | 53.7 | 52.0 | | 85.0 | 81.7 | 76.3 | 95.3 | 73.3 |
| | 25 | 0 | 81.3 | 86.0 | 82.0 | 70.7 | 61.7 | 37.3 | 86.7 | 86.7 | 80.7 | 96.0 | 72.0 |
| | 50 | 0 | 85.0 | | 77.3 | 75.7 | 59.3 | 35.0 | | | | 97.3 | 79.0 |
| | 100 | 0 | | | 82.3 | 91.0 | 78.7 | 44.3 | | | | | 86.7 |
| | 200 | 0 | | | | | 81.3 | 62.3 | | | | | 86.7 |
| | 400 | 0 | | | | | | 55.0 | | | | | 86.7 |
| | 800 | 0 | | | | | | 71.7 | | | | | |

* DAT = Days after treatment

TABLE I

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| | 0 | 0.390625 | 2.0 | 0.0 | 12.3 | 8.3 | 4.0 | | | | 0.0 | | |
| 2 above | 0 | 0.78125 | 4.0 | 0.0 | 13.3 | 13.3 | 2.0 | | | | 2.0 | | |
| | 0 | 1.5625 | 13.3 | 3.3 | 29.0 | 13.3 | 15.0 | | | | 3.3 | | |
| | 0 | 3.125 | 13.3 | 1.0 | 21.7 | 13.3 | 11.0 | 0.0 | 0.0 | 0.7 | 6.7 | 0.0 | 0.0 |
| | 0 | 6.25 | 25.0 | 1.7 | 28.3 | 16.7 | 10.0 | 0.0 | 0.0 | 0.0 | 11.7 | 0.0 | 0.0 |
| | 0 | 12.5 | 51.7 | 11.7 | 43.3 | 25.0 | 26.0 | 0.7 | 0.7 | 0.0 | 8.3 | 0.0 | 0.0 |
| | 0 | 25 | 60.7 | 30.0 | 54.0 | 38.3 | 47.3 | 0.0 | 0.0 | 0.0 | 20.0 | 0.0 | 0.0 |
| 1 + 2 | 0.390625 | 0.390625 | 5.7 | 3.7 | 14.0 | 11.7 | 1.0 | | 10.0 | 5.7 | 3.7 | | |
| in a | 0.78125 | 0.78125 | 12.3 | 10.7 | 16.7 | 18.3 | 5.0 | | 9.0 | 9.7 | 8.3 | | |
| ratio | 1.5625 | 1.5625 | 38.3 | 14.0 | 41.7 | 15.7 | 15.0 | | 40.0 | 16.3 | 26.7 | 38.3 | |
| of 1:1 | 3.125 | 3.125 | 44.0 | 25.0 | 41.7 | 24.3 | 26.7 | | 51.7 | 41.7 | 38.3 | 67.3 | |
| | 6.25 | 6.25 | 53.7 | 46.7 | 60.7 | 33.3 | 43.3 | | 85.0 | 65.0 | 59.0 | 75.7 | |
| | 12.5 | 12.5 | 76.7 | 75.7 | 70.3 | 58.3 | 55.7 | | 88.3 | 84.0 | 76.7 | 91.7 | 69.3 |
| | 25 | 25 | 80.0 | 87.3 | 79.3 | 77.7 | 76.0 | 40.7 | 90.0 | 87.3 | 80.0 | 96.0 | 72.7 |
| | 50 | 50 | | | | | | 54.0 | | | | 97.3 | 80.0 |
| | 100 | 100 | | | | | | 51.7 | | | | | 80.0 |
| | 200 | 200 | | | | | | 53.3 | | | | | 82.3 |
| | 400 | 400 | | | | | | 76.7 | | | | | 84.0 |
| | 800 | 800 | | | | | | 84.3 | | | | | |

TABLE I

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| 1 + 2 | 0.390625 | 1.1719 | 8.3 | 3.0 | 20.7 | 10.0 | 3.3 | | 10.7 | 11.7 | 2.7 | | |
| in a | 0.78125 | 2.34375 | 10.0 | 6.7 | 33.3 | 10.0 | 7.3 | | 15.0 | 12.3 | 3.0 | | |
| ratio of | 1.5625 | 4.6875 | 26.7 | 13.0 | 34.0 | 21.7 | 9.0 | | 31.7 | 24.3 | 16.7 | 35.0 | |
| 1:3 | 3.125 | 9.375 | 36.7 | 25.0 | 48.3 | 38.3 | 22.3 | | 46.7 | 55.7 | 26.0 | 72.0 | |
| | 6.25 | 18.25 | 72.3 | 57.7 | 65.0 | 51.7 | 59.0 | | 81.7 | 64.0 | 60.3 | 78.3 | |
| | 12.5 | 37.5 | 76.3 | 66.0 | 75.0 | 69.0 | 74.0 | | 88.3 | 78.0 | 65.7 | 86.7 | 70.3 |
| | 25 | 75 | | | | | | 36.7 | 90.0 | 87.7 | | 94.3 | 73.0 |
| | 50 | 150 | | | | | | 45.7 | | | | 98.0 | 74.3 |
| | 100 | 300 | | | | | | 59.3 | | | | 98.0 | 74.3 |
| | 200 | 600 | | | | | | 51.0 | | | | | 90.0 |
| | 400 | 1200 | | | | | | 80.7 | | | | | 89.0 |
| | 800 | 2400 | | | | | | 80.0 | | | | | |

TABLE I

| Compound | RATE g/ha Compound 1 | RATE g/ha Compound 2 | DG | SH | ST | AG | LL | CN | EH | PI | CA | TM | ECA |
|---|---|---|---|---|---|---|---|---|---|---|---|---|---|
| 1 + 2 | 1.1719 | 0.390625 | 17.3 | 10.0 | 21.7 | 10.0 | 15.0 | | 22.7 | 10.0 | 8.3 | | |
| in a | 2.34375 | 0.78125 | 31.7 | 14.0 | 39.0 | 21.7 | 16.7 | | 48.3 | 18.3 | 19.3 | 50.0 | |
| ratio of | 4.6875 | 1.5625 | 46.7 | 31.7 | 46.7 | 31.3 | 17.3 | | 58.3 | 58.3 | 45.0 | 66.7 | |
| 3:1 | 9.375 | 3.125 | 62.7 | 70.0 | 58.3 | 57.7 | 38.3 | | 83.3 | 71.0 | 60.0 | 81.7 | 69.3 |
| | 18.75 | 6.25 | 78.3 | 70.0 | 75.3 | 61.3. | 54.0 | | 86.7 | 83.3 | 73.7 | 95.3 | 72.0 |
| | 37.5 | 12.5 | 78.0 | 78.3 | 81.7 | 79.3 | 75.3 | 56.7 | 88.3 | 85.0 | 82.3 | 97.0 | 73.3 |
| | 75 | 25 | 93.3. | 87.0 | 91.7 | 84.3 | 84.0 | 41.7 | | | 84.0 | 98.0 | 82.7 |
| | 150 | 50 | | | | | | 52.7 | | | | | 85.0 |
| | 300 | 100 | | | | | | 70.3 | | | | | 87.3 |
| | 600 | 200 | | | | | | 80.3 | | | | | 87.7 |
| | 1200 | 400 | | | | | | 77.7 | | | | | |

TABLE I

17 DAT RESULTS

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| 1 | 0.78125 | | | 4.3 | | | | | 26.0 | 5.0 | 8.0 | | |
| above | 1.5625 | 0 | 13.3 | 7.3 | | | | | 32.3 | 12.3 | 19.0 | 27.7 | |
| | 3.125 | 0 | 32.7 | 16.0 | 31.0 | 35.0 | | | 56.7 | 50.0 | 42.3 | 51.7 | |
| | 6.25 | 0 | 46.7 | 37.3 | 50.0 | 47.3 | 21.7 | | 73.3 | 70.7 | 58.0 | 85.0 | |
| | 12.5 | 0 | 81.3 | 71.3 | 64.0 | 56.3 | 44.3 | | 86.0 | 92.7 | 75.7 | 100.0 | 55.0 |
| | 25 | 0 | 96.7 | 98.0 | 88.3 | 71.7 | 53.7 | 26.0 | 90.7 | 98.0 | 85.9 | 100.0 | 68.3 |
| | 50 | 0 | 98.3 | | 83.3 | 82.7 | 53.3 | 24.3 | | | | 100.0 | 78.0 |
| | 100 | 0 | | | 93.3 | 96.0 | 76.3 | 28.3 | | | | | 91.3 |
| | 200 | 0 | | | | | 82.7 | 46.7 | | | | | 93.3 |
| | 400 | 0 | | | | | | 52.7 | | | | | 92.3 |
| | 800 | 0 | | | | | | 76.3 | | | | | |

TABLE I

17 DAT RESULTS

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| 2 | 0 | 0.390625 | 2.7 | 1.7 | 12.3 | 10.0 | 8.3 | | | | 2.3 | | |
| above | 0 | 0.78125 | 2.3 | 0.0 | 12.3 | 10.0 | 3.3 | | | | 10.0 | | |
| | 0 | 1.5625 | 25.7 | 1.0 | 40.0 | 6.7 | 40.0 | | | | 33.3 | | |
| | 0 | 3.125 | 25.0 | 0.0 | 35.0 | 15.0 | 26.7 | 0.0 | 0.0 | 1.0 | 38.3 | 0.0 | 0.0 |
| | 0 | 6.25 | 62.3 | 0.0 | 45.7 | 11.0 | 24.0 | 0.0 | 0.0 | 0.0 | 58.3 | 0.0 | 0.0 |
| | 0 | 12.5 | 90.7 | 13.3 | 71.0 | 21.7 | 70.7 | 0.0 | 1.7 | 0.0 | 68.7 | 0.0 | 0.0 |
| | 0 | 25 | 100.0 | 79.0 | 86.7 | 81.0 | 95.0 | 0.0 | 3.3 | 0.0 | 82.3 | 0.0 | 0.0 |

TABLE I

17 DAT RESULTS

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| 1 + 2 | 0.390625 | 0.390625 | 3.3 | 3.3 | 12.3 | 10.0 | 3.3 | | 11.0 | 4.3 | 5.0 | | |
| in a | 0.78125 | 0.78125 | 8.3 | 5.7 | 13.3 | 13.3 | 5.0 | | 19.0 | 10.0 | 6.3 | | |
| ratio | 1.5625 | 1.5625 | 46.7 | 9.0 | 36.7 | 13.3 | 26.7 | | 33.3 | 23.3 | 36.0 | 16.7 | |
| of 1:1 | 3.125 | 3.125 | 55.0 | 13.0 | 38.3 | 25.0 | 31.7 | | 48.3 | 39.0 | 38.7 | 46.7 | |
| | 6.25 | 6.25 | 55.0 | 36.7 | 58.7 | 33.3 | 26.0 | | 81.7 | 63.7 | 56.7 | 62.0 | |
| | 12.5 | 12.5 | 84.0 | 77.3 | 69.7 | 55.3 | 59.7 | | 91.0 | 96.5 | 778.3 | 91.0 | 52.3 |
| | 25 | 25 | 99.7 | 99.3 | 93.3. | 82.3 | 97.0 | 26.7 | 93.3. | 93.7 | 88.3 | 98.7 | 65.0 |
| | 50 | 50 | | | | | | 26.7 | | | | 100.0 | 77.0 |
| | 100 | 100 | | | | | | 25.0 | | | | | 82.3 |
| | 200 | 200 | | | | | | 43.3 | | | | | 85.0 |
| | 400 | 400 | | | | | | 83.0 | | | | | 91.3 |
| | 800 | 800 | | | | | | 96.7 | | | | | |

**TABLE I**

17 DAT RESULTS

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| 1 + 2 | 0.390625 | 1.1719 | 28.0 | 4.0 | 25.0 | 5.0 | 11.7 | | 33.3 | 8.3 | 3.7 | | |
| in a | 0.78125 | 2.34375 | 7.3 | 5.7 | 30.0 | 11.7 | 23.3 | | 22.3 | 7.3 | 9.0 | | |
| ratio | 1.5625 | 4.6875 | 34.3 | 9.0 | 43.3 | 17.3 | 32.3 | | 31.7 | 20.0 | 34.3 | 22.7 | |
| of 1:3 | 3.125 | 9.375 | 37.7 | 16.7 | 49.0 | 35.7 | 21.7 | | 58.7 | 51.3 | 31.7 | 52.0 | |
| | 6.25 | 18.75 | 94.7 | 48.0 | 75.7 | 56.7 | 87.7 | | 67.7 | 64.3 | 79.0 | 83.3 | |
| | 12.5 | 37.5 | 99.7 | 86.7 | 81.0 | 79.7 | 96.7 | | 89.3 | 81.0 | 84.0 | 93.3 | 51.7 |
| | 25 | 75 | | | | | | 31.7 | 93.0 | 99.0 | | 100.0 | 62.3 |
| | 50 | 150 | | | | | | 25.0 | | | | 100.0 | 68.7 |
| | 100 | 300 | | | | | | 51.7 | | | | | 81.7 |
| | 200 | 600 | | | | | | 50.3 | | | | | 97.7 |
| | 400 | 1200 | | | | | | 97.0 | | | | | 98.7 |
| | 800 | 2400 | | | | | | 98.0 | | | | | |

TABLE I

17 DAT RESULTS

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| 1 + 2 | 1.1719 | 0.390625 | 12.3 | 11.3 | 16.7 | 15.0 | 13.3. | | 24.0 | 7.3 | 6.3 | | |
| in a | 2.34375 | 0.78125 | 25.7 | 12.3 | 31.7 | 20.0 | 15.7 | | 45.0 | 16.7 | 21.7 | 32.0 | |
| ratio | 4.6875 | 1.5625 | 44.0 | 18.3 | 53.3 | 37.3 | 17.3 | | 68.7 | 58.0 | 42.3 | 53.0 | |
| of 3:1 | 9.375 | 3.125 | 69.0 | 67.7 | 54.3 | 51.7 | 47.7 | | 86.0 | 70.0 | 64.0 | 78.3 | 50.0 |
| | 18.75 | 6.25 | 79.0 | 78.3 | 75.0 | 68.3 | 43.3 | | 91.0 | 95.7 | 75.7 | 100.0 | 64.0 |
| | 37.5 | 12.5 | 99.0 | 83.3 | 90.3 | 87.0 | 75.7 | 38.3 | 94.7 | 98.7 | 89.3 | 100.0 | 70.3 |
| | 75 | 25 | 100.0 | 99.0 | 98.0 | 97.7 | 98.3 | 28.3 | | | 95.0 | 100.0 | 78.0 |
| | 150 | 50 | | | | | | 42.3 | | | | | 90.7 |
| | 300 | 100 | | | | | | 56.0 | | | | | 93.0 |
| | 600 | 200 | | | | | | 84.7 | | | | | 97.3 |
| | 1200 | 400 | | | | | | 97.0 | | | | | |

TABLE I

28 DAT RESULTS

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| 1 | 0.78125 | | | 6.7 | | | | | 16.7 | 3.3 | 8.7 | | |
| above | 1.5625 | 0 | 8.3 | 7.7 | | | | | 24.0 | 4.3 | 23.3 | 17.3 | |
| | 3.125 | 0 | 15.0 | 31.0 | 21.7 | 16.7 | | | 53.3 | 35.0 | 37.7 | 30.3 | |
| | 6.25 | 0 | 31.0 | 50.0 | 40.7 | 17.3 | 13.3 | | 74.0 | 66.0 | 51.7 | 75.0 | |
| | 12.5 | 0 | 72.0 | 73.3 | 57.7 | 35.0 | 32.3 | | 86.7 | 92.3 | 78.0 | 100.0 | 60.0 |
| | 25 | 0 | 93.3. | 100.0 | 91.3 | 63.7 | 36.7 | 30.3 | 92.7 | 97.7 | 85.3 | 99.3 | 70.0 |
| | 50 | 0 | 98.7 | | 83.3 | 79.0 | 26.0 | 24.3 | | | | 100.0 | 83.3 |
| | 100 | 0 | | | 96.0 | 94.3 | 73.0 | 24.3 | | | | | 96.7 |
| | 200 | 0 | | | | | 83.7 | 31.0 | | | | | 93.3 |
| | 400 | 0 | | | | | | 52.0 | | | | | 99.3 |
| | 800 | 0 | | | | | | 85.3 | | | | | |

**TABLE I**

28 DAT RESULTS

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| 2 | | 0.390625 | 3.7 | 15.7 | 10.7 | 6.0 | 8.3 | | | | 4.3 | | |
| above | | 0.78125 | 3.3 | 3.3 | 14.0 | 1.7 | 7.3 | | | | 5.0 | | |
| | | 1.5625 | 17.7 | 11.7 | 35.0 | 5.0 | 33.7 | | | | 42.3 | | |
| | | 3.125 | 15.0 | 2.0 | 30.0 | 1.7 | 30.7 | 0.0 | 0.0 | 0.0 | 52.3 | 0.0 | 0.0 |
| | | 6.25 | 60.7 | 4.3 | 40.7 | 5.0 | 34.3 | 0.0 | 0.0 | 0.0 | 61.7 | 0.0 | 0.0 |
| | | 12.5 | 91.7 | 11.7 | 77.3 | 3.3 | 77.3 | 0.0 | 0.7 | 0.0 | 83.3 | 0.0 | 0.0 |
| | | 25 | 100.0 | 85.0 | 97.3 | 87.7 | 98.0 | 0.0 | 3.3 | 0.0 | 97.3 | 0.0 | 0.0 |

TABLE I

28 DAT RESULTS

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| 1 + 2 | 0.390625 | 0.390625 | 3.3 | 5.0 | 18.3 | 2.7 | 1.7 | | 4.0 | 3.3. | 14.0 | | |
| in a | 0.78125 | 0.78125 | 7.0 | 25.0 | 16.3 | 2.7 | 11.7 | | 22.3 | 4.3 | 11.3 | | |
| ratio | 11.5625 | 1.5625 | 44.7 | 8.3 | 38.3 | 5.0 | 24.3 | | 24.0 | 21.0 | 38.3 | 13.3 | |
| of 1:1 | 3.125 | 3.125 | 47.0 | 26.7 | 36.0 | 13.3 | 25.0 | | 47.7 | 37.3 | 31.7 | 22.7 | |
| | 6.25 | 6.25 | 41.7 | 44.3 | 46.7 | 15.0 | 15.7 | | 81.3 | 62.0 | 50.0 | 45.0 | |
| | 12.5 | 12.5 | 77.3 | 76.3 | 66.7 | 43.3 | 41.3 | | 88.3 | 98.0 | 80.0 | 88.0 | 96.7 |
| | 25 | 25 | 100.00 | 100.00 | 98.3 | 71.0 | 99.0 | 20.0 | 95.0 | 94.3 | 96.7 | 96.7 | 81.7 |
| | 50 | 50 | | | | | | 21.7 | | | | 100.0 | 68.0 |
| | 100 | 100 | | | | | | 22.7 | | | | | 84.3 |
| | 200 | 200 | | | | | | 37.7 | | | | | 90.0 |
| | 400 | 400 | | | | | | 82.3 | | | | | 100.0 |
| | 800 | 800 | | | | | | 97.0 | | | | | |

TABLE I

28 DAT RESULTS

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| 1 + 2 | 0.390625 | 1.1719 | 29.3 | 11.7 | 26.7 | 9.0 | 11.7 | | 33.7 | 10.7 | 7.3 | | |
| in a | 0.78125 | 2.3438 | 6.0 | 23.3 | 34.0 | 10.0 | 20.0 | | 23.3 | 9.0 | 9.7 | | |
| ratio | 1.5625 | 4.6876 | 22.7 | 20.0 | 38.3 | 11.7 | 26.7 | | 28.3 | 21.7 | 37.7 | 13.3 | |
| 1:3 | 3.125 | 9.3752 | 32.3 | 21.0 | 48.3 | 12.3 | 19.3 | | 62.3 | 45.0 | 21.0 | 34.3 | |
| | 6.25 | 18.7504 | 99.3 | 49.3 | 81.7 | 38.3 | 91.7 | | 65.7 | 60.0 | 90.7 | 73.7 | |
| | 12.5 | 37.5008 | 100.0 | 83.3 | 90.0 | 64.3 | 99.0 | | 93.3 | 81.3 | 95.7 | 86.3 | 62.7 |
| | 25 | 75.0016 | | | | | | 29.7 | 95.0 | 100.0 | | 100.0 | 47.3 |
| | 50 | 150.0032 | | | | | | 27.0 | | | | 100.0 | 68.3 |
| | 100 | 300.0064 | | | | | | 50.0 | | | | | 88.7 |
| | 200 | 600.0128 | | | | | | 40.0 | | | | | 100.0 |
| | 400 | 1200.025 | | | | | | 97.7 | | | | | 100.0 |
| | 800 | 2400.051 | | | | | | 98.0 | | | | | |

## TABLE I

### 28 DAT RESULTS

| Compound | RATE g/ha Compound 1 | RATE g/ha Compound 2 | DG | SH | ST | AG | LL | CN | EH | PI | EI | TM | ECA |
|---|---|---|---|---|---|---|---|---|---|---|---|---|---|
| 1 + 2 | 1.1719 | 0.390625 | 5.3 | 21.7 | 21.7 | 6.0 | 10.0 | | 22.7 | 5.0 | 15.0 | | |
| in a | 2.3438 | 0.78125 | 21.7 | 20.0 | 32.7 | 6.7 | 17.3 | | 48.3 | 24.0 | 24.3 | 17.3 | |
| ratio | 4.6876 | 11.5625 | 41.0 | 28.3 | 51.7 | 26.0 | 19.0 | | 74.0 | 47.7 | 38.3 | 38.7 | |
| 3:1 | 9.3752 | 3.125 | 67.3 | 71.7 | 49.7 | 35.7 | 38.3 | | 85.0 | 68.3 | 66.0 | 70.7 | 66.7 |
| | 18.7504 | 6.25 | 76.0 | 75.0 | 73.3 | 46.7 | 34.3 | | 91.7 | 95.0 | 74.7 | 98.7 | 48.3 |
| | 37.5008 | 12.5 | 98.7 | 83.3 | 95.7 | 79.0 | 69.3 | 32.7 | 95.0 | 100.0 | 93.3 | 100.0 | 60.3 |
| | 75.0016 | 25 | 100.0 | 100.0 | 99.7 | 98.7 | 99.0 | 28.7 | | | 99.0 | 100.0 | 68.7 |
| | 150.0032 | 50 | | | | | | 47.0 | | | | | 92.3 |
| | 300.0064 | 100 | | | | | | 60.0 | | | | | 93.3 |
| | 600.0128 | 200 | | | | | | 86.7 | | | | | 100.0 |
| | 1200.025 | 400 | | | | | | 98.7 | | | | | |

EXAMPLE 3

Compound 1 = Ethyl 2-[6-(2-chloro-6-fluoro-4-trifluoromethyl phenoxy)benzotriazol-1-yl]propionate.

Compound 3 = Iso propyl(R)-2-[4-6-7-chloro-1-oxide-1,2,4-benzotriazin-3-ylmethylaminophenoxu]-propionate.

Compounds 1 and 3 were applied in the following ratios:
0.1, 1.0, 1.1, 1.3 and 3.1 to the following established annuals:

| | |
|---|---|
| Grasses - | Eleusine indica (EI) |
| | Sorghum halepense (SH) |
| | Digitaria sanguinalis (DG) |
| | Setaria viridis (ST) |
| | Elymus repens (AG) |
| | Lolium perenne (LL) |
| Sedge - | Cyperus rotundus (CN) |
| BL Weeds - | Euphorbia heterophylla (EH) |
| | Polygonum aviculare (PI) |
| | Matricaria perforata (TM) |
| | Conyza canadensis (ECA) |

The method of Example 2 was used and damage assessed to 6, 19 and 28 days after treatment. The results are shown in Table II.

TABLE II

6 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 1 | 3.125 | | 71.7 | 73.7 | 60.0 | 38.3 | | | 85.0 | 75.7 | 56.7 | 62.0 | 40.0 |
| above | 6.25 | | 72.3 | 80.7 | 72.7 | 47.3 | 58.3 | | 88.3 | 90.7 | 67.7 | 73.3 | 60.7 |
| | 12.5 | | 78.7 | 85.0 | 84.0 | 67.3 | 57.7 | 53.3 | 88.3 | 93.3 | 76.0 | 79.3 | 58.3 |
| | 25 | | 88.3 | 93.3 | 93.3 | 78.3 | 80.0 | 74.0 | 95.0 | 96.3 | 80.0 | 86.7 | 64.0 |
| | 50 | | 90.0 | 98.0 | 98.3 | 91.0 | 88.7 | 67.3 | 95.0 | 97.7 | 82.7 | 97.0 | 80.0 |
| | 100 | | 95.7 | 97.0 | 98.3 | 98.0 | 97.3 | 77.0 | | | 91.7 | 97.3 | 80.0 |
| | 200 | | 97.7 | 90.3 | 98.7 | 98.0 | 98.0 | 68.3 | | | | | 84.0 |
| | 400 | | | | | | 98.3 | 72.7 | | | | | |
| | 800 | | | | | | | 77.7 | | | | | |

TABLE II

6 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| | | 1.5625 | 10.7 | 29.0 | 45.7 | 7.0 | 1.7 | 0.0 | | | | | |
| 3 | | 3.125 | 32.7 | 22.7 | 34.0 | 11.7 | 2.7 | 0.0 | | | | | |
| above | | 6.25 | 54.0 | 19.0 | 36.7 | 16.7 | 20.7 | 0.0 | 86.7 | 0.7 | 12.3 | 0.0 | 0.0 |
| | | 12.5 | 70.7 | 47.0 | 67.0 | 41.7 | 56.3 | 0.0 | 3.7 | 0.0 | 0.7 | 0.0 | 0.0 |
| | | 25 | 71.0 | 58.3 | 70.7 | 48.7 | 60.7 | 5.0 | 1.0 | 0.7 | 0.0 | 0.0 | 0.7 |
| | | 50 | 73.5 | 66.3 | 75.7 | 59.0 | 63.7 | 3.3 | 8.3 | 0.0 | 0.0 | 0.0 | 0.0 |
| | | 100 | 74.0 | 70.0 | 76.7 | 61.0 | 64.3 | 5.0 | 45.7 | 0.0 | 0.0 | 0.0 | 0.0 |
| | 1.5625 | 1.5625 | 78.7 | 69.0 | 56.0 | 24.0 | 35.7 | | 81.7 | 48.7 | 57.7 | 49.0 | 31.0 |
| 1+3 | 3.125 | 3.125 | 69.3 | 81.3 | 70.0 | 48.7 | 47.7 | | 85.0 | 72.3 | 59.3 | 61.7 | 31.0 |
| in a | 6.25 | 6.25 | 74.0 | 76.7 | 71.0 | 52.0 | 47.3 | | 90.0 | 88.3 | 64.3 | 74.3 | 47.7 |
| ratio | 12.5 | 12.5 | 79.0 | 91.7 | 77.3 | 59.3 | 70.7 | 61.7 | 91.7 | 90.7 | 76.3 | 78.7 | 76.0 |
| 1:1 | 25 | 25 | 92.3 | 92.3 | 93.3 | 81.0 | 77.0 | 65.7 | 95.0 | 98.0 | 87.7 | 85.0 | 76.0 |
| | 50 | 50 | 90.3 | 98.0 | 98.0 | 93.3 | 86.0 | 70.0 | 94.3 | 98.0 | 81.7 | 96.3 | 75.0 |
| | 100 | 100 | 96.0 | 98.0 | 98.7 | 97.7 | 95.0 | 65.7 | | | 92.7 | 97.0 | 82.0 |
| | 200 | 200 | | | | | | 73.7 | | | | | 81.7 |
| | 400 | 400 | | | | | | 75.7 | | | | | |
| | 800 | 800 | | | | | | 82.7 | | | | | |

TABLE II

6 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 1+3 | 1.5625 | 4.6875 | 52.7 | 56.7 | 58.3 | 28.3 | 31.7 | | 83.3 | 65.7 | 50.7 | 52.3 | |
| in a | 3.125 | 9.375 | 70.7 | 63.7 | 67.7 | 46.3 | 49.0 | | 85.0 | 73.7 | 62.3 | 65.7 | 41.0 |
| ratio | 6.25 | 18.75 | 75.0 | 72.0 | 74.0 | 60.0 | 71.0 | | 90.0 | 80.7 | 70.0 | 75.0 | 59.3 |
| 1:3 | 12.5 | 37.5 | 80.7 | 82.0 | 89.3 | 74.7 | 75.0 | 59.0 | 91.0 | 97.3 | 75.3 | 84.0 | 69.0 |
| | 25 | 75 | 94.3 | 95.0 | 92.0 | 75.7 | 75.7 | 69.0 | 92.0 | 98.0 | 82.0 | 84.3 | 71.0 |
| | 50 | 150 | 94.0 | 98.0 | 97.0 | 96.0 | 81.7 | 69.3 | 95.0 | 98.0 | 87.3 | 95.7 | 75.7 |
| | 100 | 300 | | | | | | 75.7 | | | | | 77.3 |
| | 200 | 600 | | | | | | 62.7 | | | | | 80.3 |
| | 400 | 1200 | | | | | | 73.7 | | | | | |

**TABLE II**

6 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 1+3 | 4.6875 | 1.5625 | 68.7 | 74.0 | 68.0 | 37.3 | 37.3 | | 90.0 | 72.3 | 73.3 | 62.3 | 39.3 |
| in a | 9.375 | 3.125 | 76.7 | 87.7 | 75.7 | 54.7 | 58.7 | 56.7 | 92.7 | 91.7 | 70.3 | 73.3. | 58.7 |
| ratio | 18.75 | 6.25 | 78.7 | 82.3 | 93.7 | 59.0 | 69.0 | 71.3 | 90.0 | 97.7 | 85.3 | 81.0 | 61.7 |
| | 37.5 | 12.5 | 89.7 | 97.3 | 97.3 | 82.7 | 84.3 | 62.7 | 95.0 | 98.0 | 78.0 | 88.0 | 72.7 |
| | 75 | 25 | 93.7 | 92.0 | 98.3 | 92.0 | 86.3 | 71.7 | 95.0 | 98.0 | 86.0 | 96.7 | 75.7 |
| | 150 | 50 | 95.7 | 97.3 | 98.7 | 96.3 | 97.0 | 69.7 | | 98.0 | 90.7 | 97.3 | 84.7 |
| | 300 | 100 | | | | | | 74.3 | | | | | 81.7 |
| | 600 | 200 | | | | | | 86.3 | | | | | |

TABLE II

19 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 1 | 3.125 | | 53.7 | | 18.3 | 35.0 | | | | 70.0 | 38.3 | 15.0 | 21.7 |
| above | 6.25 | | 65.7 | 92.0 | 53.3 | 46.7 | 28.3 | | 92.7 | | 60.7 | 48.0 | 35.0 |
| | 12.5 | | | 96.7 | 74.0 | 55.0 | 23.3 | 42.7 | 90.0 | 98.7 | 68.3 | 62.7 | 36.0 |
| | 25 | | 96.7 | 100.0 | | 69.7 | | 71.0 | 94.7 | 97.3 | | | 53.3 |
| | 50 | | 96.7 | 100.0 | 99.0 | | 90.3 | 42.3 | 95.0 | 99.7 | 99.0 | 99.0 | 74.0 |
| | 100 | | 100.0 | 100.0 | 99.0 | 98.7 | 99.0 | 73.3 | | | 99.0 | 99.0 | 79.0 |
| | 200 | | 100.0 | 98.7 | 99.0 | 98.3 | 99.0 | 50.7 | | | | | |
| | 400 | | | | | | | 71.0 | | | | | |
| | 800 | | | | | | | | | | | | |

TABLE II

19 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 3 | | 1.5625 | 7.3 | 50.0 | 47.0 | 3.3 | 1.7 | 0.0 | | | | | |
| above | | 3.125 | 25.7 | 34.3 | 21.7 | 7.7 | 0.0 | 0.0 | | | | | |
| | | 6.25 | 64.7 | 72.0 | 45.7 | 31.7 | 5.7 | 0.0 | 87.7 | 1.7 | 0.0 | 3.3 | 2.5 |
| | | 12.5 | 100.0 | 91.7 | 92.0 | 80.7 | 76.0 | 0.0 | 7.3 | 1.0 | 1.7 | 1.7 | 1.7 |
| | | 25 | 98.7 | 100.0 | 97.3 | 87.7 | 87.3 | 3.3 | 8.3 | 4.3 | 1.7 | 1.7 | 3.3 |
| | | 50 | 100.0 | 100.0 | 99.0 | 93.0 | 92.3 | 10.0 | 17.0 | 5.3 | 10.0 | 1.7 | 1.7 |
| | | 100 | 99.3 | 99.7 | 99.0 | 94.3 | 96.0 | 0.0 | 65.0 | 5.3 | 3.3 | 5.7 | 2.7 |

TABLE II

19 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 1+3 | 1.5625 | 1.5625 | 81.3 | 73.3 | 8.3 | 35.0 | 4.3 | | 80.3 | 42.7 | 31.7 | 15.7 | 15.7 |
| | 3.125 | 3.125 | 52.0 | 88.7 | 40.0 | 44.0 | 7.7 | | 88.3 | 71.7 | 43.3 | | |
| in a | 6.25 | 6.25 | 65.7 | 83.7 | 46.0 | 38.3 | 6.7 | | 87.3 | 98.0 | 60.0 | 30.0 | 28.3 |
| ratio | 12.5 | 12.5 | 89.7 | 96.7 | 58.0 | 53.3 | 39.3 | 37.3 | 94.3 | 97.7 | 77.3 | 73.0 | 49.0 |
| 1:1 | 25 | 25 | 100.0 | 97.7 | 92.0 | 97.3 | 76.0 | 53.3 | 93.0 | 99.3 | 98.3 | 84.3 | 60.7 |
| | 50 | 50 | 99.7 | 100.0 | 99.0 | 98.7 | 89.7 | 52.7 | 94.0 | 99.7 | 98.7 | 99.0 | 68.3 |
| | 100 | 100 | 100.0 | 100.0 | 99.0 | 98.7 | 98.0 | 50.0 | | | 98.7 | 99.0 | 78.0 |
| | 200 | 200 | | | | | | 57.7 | | | | | 78.3 |
| | 400 | 400 | | | | | | 64.3 | | | | | |
| | 800 | 800 | | | | | | | | | | | |

TABLE II

19 DAT RESULTS

| Compound | RATE g/ha Compound 1 | RATE g/ha Compound 2 | DG | SH | ST | AG | LL | CN | EH | PI | CA | TM | ECA |
|---|---|---|---|---|---|---|---|---|---|---|---|---|---|
| 1 + 3 | 1.5625 | 4.6875 | 25.0 | 54.0 | 12.3 | 30.7 | 1.7 | | 81.7 | 66.7 | 37.7 | 12.0 | |
| in a | 3.125 | 9.375 | 57.0 | 66.7 | 37.3 | 48.3 | 8.3 | | 88.7 | 74.7 | 43.3 | 16.0 | 22.7 |
| ratio | 6.25 | 18.75 | 76.3 | 78.3 | 56.0 | 63.3 | 53.7 | | 92.0 | 80.0 | 67.3 | 41.7 | 30.0 |
| 1:3 | 12.5 | 37.5 | 97.0 | 99.7 | 98.7 | 90.7 | 84.7 | 39.0 | 92.7 | 99.7 | 82.3 | 76.7 | 35.0 |
| | 25 | 75 | 100.0 | 100.0 | 92.7 | 88.7 | 81.7 | 58.3 | 92.7 | | 89.3 | 77.7 | 50.0 |
| | 50 | 150 | 100.0 | 100.0 | 99.0 | 94.3 | 96.7 | 60.0 | 94.0 | 99.7 | 99.0 | 98.7 | 63.0 |
| | 100 | 300 | | | | | | 70.0 | | | | | 68.3 |
| | 200 | 600 | | | | | | 58.3 | | | | | 77.3 |
| | 400 | 1200 | | | | | | 60.7 | | | | | |

TABLE II

19 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 1 + 3 | 4.6875 | 1.5625 | 31.7 | 87.3 | 38.3 | 41.7 | 2.7 | | 92.3 | 75.7 | 65.0 | 18.3 | 24.3 |
| in a | 9.375 | 3.125 | 59.3 | 96.3 | 64.3 | 45.0 | 29.3 | 45.0 | 93.3 | 99.3 | 63.3 | 42.3 | 30.0 |
| ratio | 18.75 | 6.25 | 83.3 | 92.3 | 99.0 | 53.3 | 37.3 | 60.0 | 91.0 | 99.3 | 97.0 | 77.7 | 42.7 |
| 3:1 | 37.5 | 12.5 | 94.7 | 100.0 | 99.0 | 84.3 | 86.3 | 46.7 | 95.0 | 100.0 | 90.3 | 92.3 | 65.7 |
| | 75 | 25 | 100.0 | 98.3 | 99.0 | 96.3 | 89.3 | 58.3 | 94.3 | 100.0 | 99.0 | 98.7 | 70.7 |
| | 150 | 50 | 100.0 | 99.7 | 99.0 | 98.3 | 99.0 | 56.7 | | 99.7 | 99.0 | | 83.0 |
| | 300 | 100 | | | | | | 56.7 | | | | | 86.7 |
| | 600 | 200 | | | | | | 72.3 | | | | | |

TABLE II

28 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 1 | 3.125 | | 43.3 | 81.3 | 25.0 | 21.7 | | | 90.7 | 61.7 | 30.0 | 2.7 | 31.7 |
| above | 6.25 | 0 | 55.0 | 89.3 | 59.3 | 35.0 | 11.7 | | 92.7 | 97.0 | 48.3 | 23.7 | 47.7 |
| | 12.5 | 0 | 71.7 | 97.3 | 75.0 | 49.0 | 15.0 | 38.3 | 92.3 | 99.0 | 59.0 | 32.7 | 45.7 |
| | 25 | 0 | 95.0 | 100.0 | 100.0 | 64.3 | 78.7 | 61.0 | 94.3 | 96.0 | 90.7 | 90.7 | 53.7 |
| | 50 | 0 | 97.0 | 100.0 | 100.0 | 87.7 | 86.7 | 42.7 | 94.3 | 99.7 | 100.0 | 99.3 | 86.0 |
| | 100 | 0 | 100.0 | 100.0 | 100.0 | 99.3 | 99.0 | 59.7 | | | 100.0 | 100.0 | 90.0 |
| | 200 | 0 | 100.0 | 98.7 | 100.0 | 99.3 | 99.0 | 45.0 | | | | | 89.3 |
| | 400 | 0 | | | | | 99.0 | 72.0 | | | | | |
| | 800 | 0 | | | | | | 81.7 | | | | | |

TABLE II

28 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 3 | 0 | 1.5625 | 0.3 | 55.0 | 51.0 | 0.0 | 4.0 | 0.0 | | | | | |
| above | 0 | 3.125 | 15.3 | 34.3 | 18.3 | 3.3 | 0.0 | 10.0 | | | | | |
| | 0 | 6.25 | 55.7 | 72.0 | 58.7 | 20.0 | 12.7 | 0.0 | | 0.7 | 12.0 | 4.0 | 11.7 |
| | 0 | 12.5 | 100.0 | 88.7 | 98.3 | 77.0 | 84.3 | 0.0 | 5.0 | 0.0 | 0.0 | 1.7 | 0.0 |
| | 0 | 25 | 100.0 | 100.0 | 99.0 | 91.7 | 93.7 | 0.0 | 4.0 | 1.7 | 0.0 | 1.0 | 0.0 |
| | 0 | 50 | 100.0 | 100.0 | 100.0 | 98.0 | 97.0 | 0.0 | 6.7 | 4.3 | 18.0 | 4.3 | 0.0 |
| | 0 | 100 | 100.0 | 100.0 | 100.0 | 98.0 | 98.7 | 10.0 | 54.0 | 3.3 | 6.7 | 145.0 | 0.0 |

**TABLE II**

28 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 1 + 2 | 1.5625 | 1.5625 | 67.7 | 65.3 | 5.0 | 28.3 | 1.7 | | 75.7 | 38.3 | 27.7 | 3.7 | 30.0 |
| in a | 3.125 | 3.125 | 29.0 | 85.7 | 49.0 | 35.0 | 3.3 | | 90.7 | 63.3 | 36.7 | 21.7 | 28.3 |
| ratio | 6.25 | 6.25 | 43.7 | 76.7 | 40.0 | 44.3 | 3.3 | | 84.3 | 98.0 | 50.0 | 23.3 | 39.3 |
| 1:1 | 12.5 | 12.5 | 76.7 | 93.3 | 60.7 | 53.7 | 23.3 | 31.7 | 92.0 | 94.7 | 71.0 | 67.0 | 52.3 |
| | 25 | 25 | 100.0 | 98.7 | 91.7 | 89.0 | 62.0 | 46.7 | 93.3 | 100.0 | 99.3 | 84.3 | 57.7 |
| | 50 | 50 | 100.0 | 100.0 | 100.0 | 99.0 | 90.7 | 47.3 | 93.0 | 100.0 | 98.7 | 100.0 | 86.7 |
| | 100 | 100 | 100.00 | 100.0 | 100.0 | 99.0 | 98.3 | 45.0 | | | 99.3 | 100.0 | 81.3 |
| | 200 | 200 | | | | | | 45.7 | | | | | 94.7 |
| | 400 | 400 | | | | | | 54.7 | | | | | |
| | 800 | 800 | | | | | | 80.0 | | | | | |

TABLE II

28 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 1+3 | 1.5625 | 4.625 | 17.3 | 49.3 | 15.0 | 28.3 | 0.7 | | 76.0 | 56.0 | 40.0 | 16.0 | |
| in a | 3.125 | 9.375 | 45.0 | 56.0 | 39.0 | 40.0 | 3.7 | | 86.7 | 65.7 | 34.3 | 16.7 | 42.3 |
| ratio | 6.25 | 18.75 | 66.3 | 65.0 | 56.0 | 61.7 | 41.7 | | 92.3 | 74.7 | 53.0 | 16.7 | 56.7 |
| 1:3 | 12.5 | 37.5 | 98.3 | 100.0 | 99.3 | 89.7 | 91.0 | 35.0 | 92.3 | 99.3 | 72.3 | 72.3 | 72.3 |
| | 25 | 75 | 100.0 | 100.0 | 92.3 | 86.3 | 79.3 | 49.7 | 92.7 | 100.0 | 85.7 | 68.7 | 59.7 |
| | 50 | 150 | 100.0 | 100.0 | 100.0 | 97.7 | 99.0 | 51.7 | 94.3 | 100.0 | 99.7 | 100.0 | 91.7 |
| | 100 | 300 | | | | | | 61.0 | | | | | 74.0 |
| | 200 | 600 | | | | | | 42.3 | | | | | 86.7 |
| | 400 | 1200 | | | | | | 45.7 | | | | | |

**TABLE II**

28 DAT RESULTS

| Compound | RATE g/ha Compound 1 | RATE g/ha Compound 3 | DG | SH | ST | AG | LL | CN | EH | PI | CA | TM | ECA |
|---|---|---|---|---|---|---|---|---|---|---|---|---|---|
| 1+3 | 4.625 | 1.5625 | 26.7 | 77.7 | 36.7 | 28.3 | 0.0 | | 91.7 | 71.0 | 58.3 | 24.3 | 35.0 |
| in a | 9.375 | 3.125 | 56.7 | 97.0 | 57.3 | 38.3 | 21.0 | 36.7 | 94.3 | 99.3 | 66.0 | 30.7 | 33.3 |
| ratio | 18.75 | 6.25 | 74.0 | 90.0 | 100.0 | 41.7 | 22.7 | 48.3 | 90.0 | 99.7 | 91.0 | 74.0 | 53.0 |
| 3:1 | 37.5 | 12.5 | 95.7 | 100.0 | 100.0 | 79.7 | 87.7 | 41.7 | 93.3 | 100.0 | 89.0 | 94.0 | 71.3 |
| | 75 | 25 | 100.0 | 98.3 | 100.0 | 87.3 | 89.7 | 55.3 | 93.3 | 99.7 | 99.3 | 100.0 | 85.0 |
| | 150 | 50 | 100.0 | 100.0 | 100.0 | 99.3 | 99.0 | 51.0 | | 100.0 | 100.0 | 100.0 | 90.0 |
| | 300 | 100 | | | | | | 53.7 | | | | | 100.0 |
| | 600 | 200 | | | | | | 64.0 | | | | | |

The results of the tests described in Examples 2 and 3 demonstrate that in the majority of cases, the treatment according to the invention produces complementarity of effect, and in some cases, even synergy is observed. This finding is unexpected since it is generally accepted that a fast acting herbicide compound (such as the compounds of formula (I)) destroys the phloem loading and hence the translocation of a systemic herbicide (such as the compounds of formula (II) or (III)) which leads to significant antagonism in

the observed effect when such components are applied together.

In fact what is found is by adding a component of formula (I), a rapid scorch (see 6DAT data) results which is not seen with the compound of formula (II) or (III) above, a feature much favoured by farmers. However, this effect is obtained without detriment to the long term effects (see 28 DAT data) of the compounds of formula (II) and (III).

Additionally one can achieve control of both broad-leaved weeds and grasses with such treatment.

## Claims

1. A method of killing or controlling unwanted plant growth which method comprises applying to the plant or to the locus thereof a compound of formula (I):

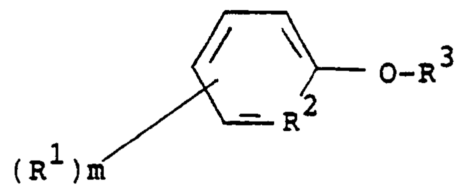

in which $R^1$ is independently selected from H, CN, $NO_2$, halogen, lower alkyl, lower haloalkyl; m is an integer of from 1 to 4; $R^2$ is N or $CR^1$ where $R^1$ is as defined above; $R^3$ is a group of formula (a), (b) or (c):

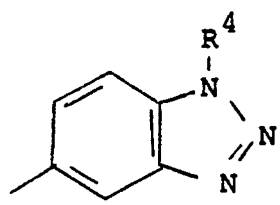

(a)

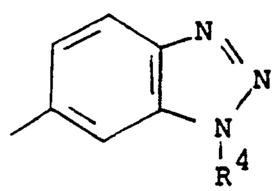

(b)

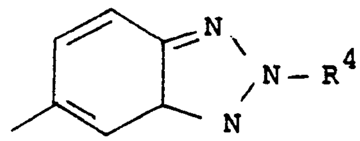

(c)

or N-oxide or quaternary salt derivatives thereof wherein $R^4$ is a group

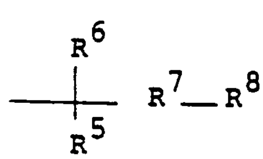

where $R^5$, $R^6$ are selected independently from H, lower alkyl, halogen or $R^5$ and $R^6$ together form $=CH_2$ or $R^5$, $R^6$ and the carbon atom to which they are attached together form a $C_{3-6}$ cycloalkyl ring;
$R^7$ is $(CH_2)_n$,

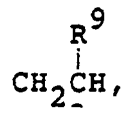

CH=CH,

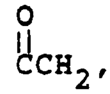

$CHOHCH_2$ or
$CHOR^{10}CH_2$ where $R^9$ is lower alkyl and $R^{10}$ is lower alkyl or phenyl; and n is 0, 1 or 2;
$R^8$ is $COOR^{11}$, OH, $OR^{10}$,

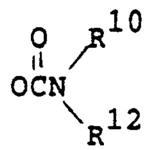 , 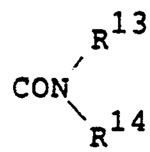 ,

$COSR^{11}$
CHO, CN, $CH=NOR^{12}$,

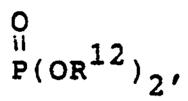

$OSO_3H$, $SO_3H$; where
$R^{10}$ is as defined hereinbefore;
$R^{11}$ is hydrogen, optionally substituted lower alkyl, optionally substituted lower alkenyl, optionally substituted lower alkynyl, $[(CH_2)_2O]_p(CH_2)_q\ OR^{10}$, $-N=C(R^{10})_2$, optionally substituted phenyl, optionally substituted benzyl, optionally substituted cycloalkyl; p is 0, 1 or 2; q is an integer from 2 to 5 inclusive;
$R^{12}$ is H or lower alkyl;
$R^{13}$ and $R^{14}$ are selected independently from H, lower alkyl, lower cycloalkyl, alkenyl, alkynyl, $SO_2R^{10}$, $N(R^{12})_2$, $^+N(R^{10})_3$; additionally $R^{14}$ may be an optionally hydroxy- or phenyl-substituted alkyl radical of 1 to 4 carbon atoms, a phenyl or chlorophenyl radical, an alkoxy radical of 1 to 4 carbon atoms, or a group $-NR^{15}R^{16}$ wherein $R^{15}$ is hydrogen or alkyl of 1 to 4 carbon atoms and $R^{16}$ is hydrogen, alkyl of 1 to 4 carbon atoms, phenyl, or chlorophenyl, or the group $-NR^{13}\ R^{14}$ constitutes a 5 or 6-membered heterocyclic ring or alkyl quaternary derivatives; and, in the case of compounds wherein $R^8$ is a carboxyl group, salts thereof;
and a compound of formula (II):

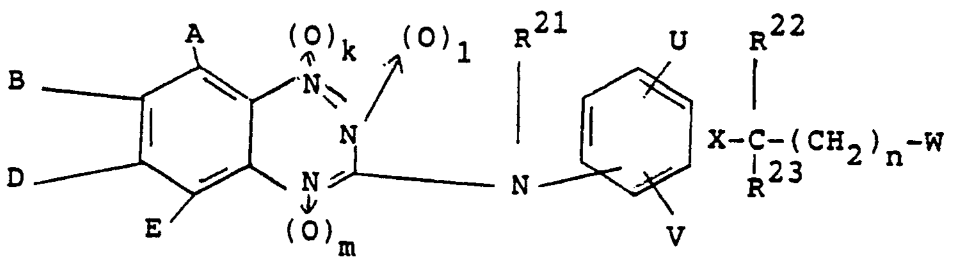 (II)

or a salt thereof wherein:
A,B,D,E,U and V are independently chosen from the group consisting of hydrogen, halogen, nitro, cyano, thiocyano, amino, $C_1$ to $C_6$ alkylamino, di($C_1$ to $C_6$ alkyl)amino, $C_1$ to $C_6$ alkyl, $C_1$ to $C_6$

haloalkyl, $C_2$ to $C_6$ alkenyl, $C_3$ to $C_7$ cycloalkyl, $C_1$ to $C_6$ alkoxy, $C_1$ to $C_6$ haloalkoxy, $C_1$ to $C_6$ alkylthio, $C_1$ to $C_6$ alkylsulfinyl, $C_1$ to $C_6$ alkylsulfonyl, $C_1$ to $C_6$ haloalkylsulfinyl, $C_1$ to $C_6$ haloalkylsulfonyl, sulfo, $C_1$ to $C_6$ alkoxysulfonyl, sulfamoyl, N-($C_1$ to $C_6$ alkyl)sulfamoyl, N-N-di($C_1$ to $C_6$ alkyl)-sulfamoyl, carboxy, ($C_1$ to $C_6$ alkoxy)-carbonyl, carbamoyl, N-($C_1$ to $C_6$ alkyl) carbamoyl, N-N di($C_1$ to $C_6$ alkyl)carbamoyl, phenyl, phenoxy, phenylthio, and the groups substituted phenyl, substituted phenoxy and substituted phenylthio wherein in each group the phenyl ring is substituted with from 1 to 3 substituents chosen from the group consisting of halogen, $C_1$ to $C_6$ alkyl, $C_1$ to $C_6$ haloalkyl, $C_1$ to $C_6$ alkoxy, nitro and cyano;

$R^{21}$ is chosen from the group consisting of hydrogen, $C_1$ to $C_{10}$ alkyl, $C_2$ to $C_{10}$ alkenyl, $C_2$ to $C_{10}$ alkynyl, $C_2$ to $C_{10}$ alkoxyalkyl, cyanomethylene, ($C_1$ to $C_6$ alkoxy)-carbonylmethylene, $C_1$ to $C_{10}$ haloalkyl, formyl, $C_2$ to $C_{10}$ alkanoyl, phenyl, benzyl, benzoyl, and the groups phenyl, benzyl and benzoyl wherein in each group the phenyl ring is substituted with from 1 to 3 substituents chosen from the group consisting of halogen, $C_1$ to $C_6$ alkyl, $C_1$ to $C_6$ haloalkyl, $C_1$ to $C_6$ alkoxy, nitro and cyano;

$R_2{}^2$ is chosen from the group consisting of hydrogen, $C_1$ to $C_6$ alkyl, $C_2$ to $C_6$ alkenyl, $C_2$ to $C_6$ alkoxyalkyl, $C_1$ to $C_6$ haloalkyl, acetyl, propionyl and $C_2$ to $C_6$ alkoxycarbonyl;

$R^{23}$ is chosen from the group consisting of hydrogen, $C_1$ to $C_6$ alkyl, $C_2$ to $C_6$ alkenyl, $C_2$ to $C_6$ alkoxyalkyl and $C_1$ to $C_6$ haloalkyl, or $R^2$ and $R^3$ together may form a methylene, ethylidene, propylidene or isopropylidene group;

W is chosen from the group consisting of cyano, thiocarbamoyl,

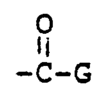

and $CH_2Z$ wherein: G is chosen from the group consisting of hydroxy, mercapto, $C_1$ to $C_{10}$ alkoxy, $C_1$ to $C_{10}$ haloalkoxy, $C_2$ to $C_{10}$ alkenyloxy, $C_2$ to $C_{10}$ alkynyloxy, $C_1$ to $C_{10}$ alkylthio, $C_2$ to $C_{10}$ alkenylthio, $C_2$ to $C_{10}$ alkynylthio, $C_3$ to $C_7$ cycloalkoxy, $C_3$ to $C_7$ cycloalkoxy substituted with 1 or 2 $C_1$ to $C_4$ alkyl groups, phenoxy, phenylthio, benzyloxy, benzylthio, the group $C_1$ to $C_6$ alkoxy substituted with a substituent chosen from the group consisting of $C_1$ to $C_6$ alkoxy, amino, ammonio, cyano, N-($C_1$ to $C_6$ alkyl)amino, N,N-di($C_1$ to $C_6$ alkyl)-amino and N,N,N-tri-($C_1$ to $C_6$ alkyl) ammonio, the groups phenoxy, phenylthio, benzyloxy and benzylthio wherein in each group the phenyl ring is substituted with from 1 to 3 substituents chosen from the group consisting of halogen, nitro, cyano, $C_1$ to $C_6$ alkyl, $C_1$ to $C_6$ haloalkyl and $C_1$ to $C_6$ alkoxy, the group OM wherein M is the cation of an inorganic or organic base, the group $-NHSO_2R^{24}$ wherein $R^{24}$ is chosen from $C_1$ to $C_{10}$ alkyl and $C_{16}$ to $C_{10}$ haloalkyl, and the group $-NR^{25}R^{26}$ wherein $R^{25}$ and $R^{26}$ are independently chosen from the group consisting of hydrogen, $C_1$ to $C_6$ alkyl, $C_1$ to $C_6$ hydroxyalkyl, $C_1$ to $C_6$ haloalkyl, phenyl and benzyl, or $R^{25}$ and $R^{26}$ together form a heterocyclic ring, and the group-O-N = $R^{27}$ wherein $R^7$ is a $C_1$ to $C_{10}$ alkylidene group; and z is chosen from halogen, hydroxy, mercapto, $C_1$ to $C_{10}$ alkoxy, $C_1$ to $C_{10}$ haloalkoxy, $C_1$ to $C_{10}$ alkylthio, and the group $NR^{25}R^{26}$ wherein $R^{25}$ and $R^{26}$ are as hereinbefore defined;

X is chosen from oxygen and sulfur;

k, l and m are independently chosen from 0 and 1 provided that k + l + m is 0, 1 or 2; and n is 0, 1 or 2.

or a compound of formula (III)

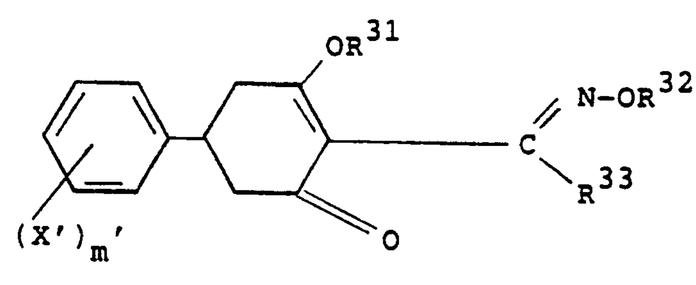

(III)

wherein:

X', which may be the same or different, are selected from the group consisting of: halogen; nitro; cyano;

$C_1$ to $C_6$ alkyl; $C_1$ to $C_6$ alkyl substituted with a substituent selected from the group consisting of

halogen, nitro, hydroxy, $C_1$ to $C_6$ alkoxy and $C_1$ to $C_6$alkylthio; $C_2$ to $C_6$ alkenyl; $C_2$ to $C_6$ alkynyl; hydroxy; $C_1$ to $C_6$ alkoxy; $C_1$ to $C_6$ alkoxy substituted with a substituent selected from halogen and $C_1$ to $C_6$ alkoxy; $C_2$ to $C_6$ alkenyloxy; $C_2$ to $C_6$ alkynyloxy; $C_2$ to $C_6$ alkanoyloxy; ($C_1$ to $C_6$ alkoxy)-carbonyl; $C_1$ to $C_6$ alkylthio; $C_1$ to $C_6$ alkylsulfinyl; $C_1$ to $C_6$ alkylsulfonyl; sulfamoyl; N-($C_1$ to $C_6$ alkyl)-sulfamoyl; N,N-di($C_1$ to $C_6$ alkyl)sulfamoyl;

benzyloxy; substituted benzyloxy wherein the benzene ring is substituted with from one to three substituents selected from the group consisting of halogen, nitro, $C_1$ to $C_6$ alkyl, $C_1$ to $C_6$ alkoxy and $C_1$ to $C_6$ haloalkyl; the group $NR^{38}$ $R^{39}$ wherein $R^{38}$ and $R^{39}$ are independently selected from the group consisting of hydrogen, $C_1$ to $C_6$ alkyl, $C_2$ to $C_6$ alkanoyl, benzoyl and benzyl; the groups formyl and $C_2$ to $C_6$ alkanoyl and the oxime, imine and Schiff base derivatives thereof; and at least one of X is not selected from the group consisting of halogen, $C_1$ to $C_6$ alkyl and $C_1$ to $C_6$ alkoxy;

$R^{31}$ is selected from the group consisting of:

hydrogen; $C_1$ to $C_6$ alkyl; $C_2$ to $C_6$ alkenyl; $C_2$ to $C_6$ alkynyl; substituted $C_1$ to $C_6$ alkyl wherein the alkyl group is substituted with a substituent from the group consisting of $C_1$ to $C_6$ alkoxy, $C_1$ to $C_6$ alkylthio, phenyl and substituted phenyl wherein the benzene ring is substituted with from one to three substituents selected from the group consisting of halogen, nitro, cyano, $C_1$ to $C_6$ alkyl, $C_1$ to $C_6$ haloalkyl, $C_1$ to $C_6$ alkoxy and $C_1$ to $C_6$ alkylthio; $C_1$ to $C_6$ (alkyl) sulfonyl; benzenesulfonyl; substituted benzenesulfonyl wherein the benzene ring is substituted with from one to three substituents selected from the group consisting of halogen, nitro, cyano, $C_1$ to $C_6$ alkyl, $C_1$ to $C_6$ haloalkyl, $C_1$ to $C_6$alkoxy and $C_1$ to $C_6$ alkylthio; and acyl group; and an inorganic or organic cation;

$R^{32}$ is selected from the group consisting of: $C_1$ to $C_6$ alkyl; $C_2$ to $C_6$ alkenyl; $C_2$ to $C_6$ haloalkenyl; $C_2$ to $C_6$ alkynyl; $C_2$ to $C_6$ haloalkynyl; substituted $C_1$ to $C_6$ alkyl wherein the alkyl group is substituted with a substituent selected from the group consisting of halogen, $C_1$ to $C_6$ alkoxy, $C_1$ to $C_6$ alkylthio, phenyl and substituted phenyl wherein the benzene ring is substituted with from one to three substituents selected from the group consisting of halogen, nitro, cyano, $C_1$ to $C_6$ alkyl, $C_1$ to $C_6$ haloalkyl, $C_1$ to $C_6$ alkoxy and $C_1$ to $C_6$ alkylthio;

$R^{37}$ is selected from the group consisting of: $C_1$ to $C_6$ alkyl; $C_1$ to $C_6$ fluoroalkyl; $C_2$ to $C_6$ alkenyl; $C_2$ to $C_6$ alkynyl; and phenyl; and

m is an integer from 3 to 5.

**2.** A method according to claim 1 where the compound of formula (I) is selected from compounds where $(R^1)_m$ isindependently selected from hydrogen, halo, nitro, cyano or haloalkyl and m is an integer of 1 to 4: $R^2$ is $C-R^1$ where $R^1$ is hydrogen, halo, nitro, cyano or haloalkyl; $R^3$ is a group of formula (b) or (c) as defined in claim 1; and $R^4$ is

$$\begin{matrix} CHR^7-R^8 \\ R^6 \end{matrix}$$

where $R^6$ is hydrogen, $C_{1-4}$ alkyl or halo, $R^7$ is $(CH_2)_n$ where n is 0, 1 or 2 and $R^8$ is a groyp $CO_2R^{11}$ where $R^{11}$ is as defined in claim 1.

**3.** A method according to claim 2 wherein $R^{11}$ is $C_{1-4}$ alkyl.

**4.** A method according to claim 2 or claim 3 wherein n is 0.

**5.** A method according to any one of the preceding claims wherein the compound of formula (II) is selected from compounds where A, D and E are hydrogen. B is halogen, k is 1 and l and m are 0, $R^{21}$ is lower alkyl, U and V are hydrogen, X is oxygen, $R^{22}$ and $R^{23}$ are independently selected from hydrogen or lower

alkyl, n is O and W is a group of

$$\begin{matrix} O \\ \| \\ -C-G \end{matrix}$$

where G is $C_1$ to $C_{10}$ alkoxy.

6. A method according to any one of claims 1 to 4 where the compound of formula (III) is selected from a compound of formula (IIIA)

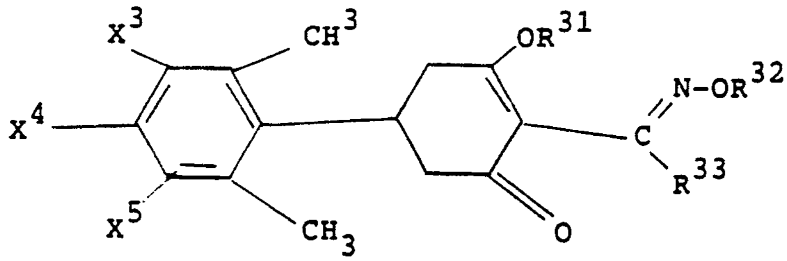

(IIIA)

wherein:
$X^3$ is selected from the group consisting of methylmercapto, nitromethyl, methoxymethyl, ethoxymethyl, methylsulfinyl, methylsulfonyl, acetyl, propionyl, sulfamoyl and N,N-dimethylsulfamoyl;
$X^4$ is selected from hydrogen and methyl;
$X^5$ is selected from hydrogen, methyl and ethyl;
$R^{31}$ is selected from hydrogen, sodium and potassium;
$R^{32}$ is selected from ethyl and allyl; and
$R^{33}$ is selected from ethyl and n-propyl.

7. A herbicidal composition comprising a compound of formula (I) as defined in claim 1, compound of formula (II) as defined in claim 1 or a compound of formula (III) as defined in claim 1, in combination with a diluent or carrier.

8. A two pack-container having a first compartment containing a composition comprising a compound of formula (I) as defined in claim 1 in combination with a solid or liquid diluent and a second compartment containing a composition comprising a compound of formula (II) or formula (III) as defined in claim 1 in combination with a solid or liquid diluent.

European Patent Office

# EUROPEAN SEARCH REPORT

Application Number

**EP 91 30 0291**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,P,D | EP-A-0 355 049 (IMPERIAL CHEMICAL INDUSTRIES)<br>- - - - - | | A 01 N 43/647 //<br>(A 01 N 43/647<br>A 01 N 43:707<br>A 01 N 35:10 ) |
| | | | **TECHNICAL FIELDS SEARCHED (Int. Cl.5)** |
| | | | A 01 N |

The present search report has been drawn up for all claims

| Place of search | Date of completion of search | Examiner |
|---|---|---|
| The Hague | 27 May 91 | DECORTE D. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document